# EUROPEAN PATENT SPECIFICATION

(11) **EP 3 398 657 B1**
(45) Date of publication and mention of the grant of the patent: **21.04.2021**
(21) Application number: 16882126.2
(22) Date of filing: 29.12.2016
(51) Int. Cl.: A62B 18/00, A41D 13/11, A62B 18/02

(54) **HARMFUL-SUBSTANCE-BLOCKING HEALTH MASK USING AIR CURTAIN**
SCHADSTOFFBLOCKIERENDE GESUNDHEITSMASKE MIT LUFTVORHANG
MASQUE HYGIÉNIQUE DE BLOCAGE DE SUBSTANCE NOCIVE UTILISANT UN RIDEAU D'AIR

(30) Priority: 29.12.2015 KR 20150189021; 28.12.2016 KR 20160181174
(43) Date of publication of application: 07.11.2018
(73) Proprietor: Han, Ga Hyun, Yuseong-gu, Daejeon 34140 (KR)
(72) Inventor: Han, Ga Hyun, Yuseong-gu, Daejeon 34140 (KR)
(74) Representative: Verscht, Thomas Kurt Albert
(86) International application number: PCT/KR2016/015495
(87) International publication number: WO 2017/116174

(56) References cited:
- EP-A1- 0 225 744
- JP-A- 2014 061 442
- JP-A- 2015 501 190
- JP-A- 2015 505 687
- KR-A- 20110 005 382
- KR-A- 20150 041 088
- KR-B1- 101 103 516
- KR-B1- 101 460 942
- US-A- 4 011 865
- US-A- 4 258 710

## Description

### Technical Field

The present invention relates to a health mask. More particularly, the present invention relates to a health mask being excellent in tight fitting to a user' s skin and configured to form an air curtain around a user's chin to block entry of external harmful substances, thereby protecting the respiratory organs of a user.

### Background Art

Unlike in the past, people in modern times are threatened by respiratory health risks because of various kinds of harmful gas, dust, fine dust, and the like. Since the advent of the industrial age, people have been at risk of suffering respiratory problems, and air pollution is becoming more intense in proportion to industrialization and urbanization.

These causes of air pollution may be classified into natural pollutants from desert sand storms, volcanic eruptions, and the like, and anthropogenic pollutants such as waste gas from plants, combustion of fossil fuels, and the like

Normally, the natural air pollutants are produced as a result of continuous or temporary natural events, which do not constitute a continuous threat to human respiratory health. On the other hand, the anthropogenic pollutants are produced as a result of human activities such as burning of fossil fuels, nuclear energy generation using nuclear power, chemical reactions, physical processes, and vehicular emissions from automobiles, aircrafts, and the like. Various pollutants produced during the combustion fossil fuels are the main cause of air pollution and have adverse effects on human respiratory health.

Of these, carbon monoxide (CO), nitrogen dioxide (NO₂), sulfur dioxide (SO₂), and the like constitute the majority of pollutants. In particular, sulfur dioxide (SO₂) reacts with other air pollutants to produce additional secondary pollutants. Such air pollutants are categorized into gaseous substances such as sulfur dioxide or carbon monoxide and particulate substances such as dust, and the like depending on particle size.

Furthermore, depending on a generation process, air pollutants fall into two categories: primary and secondary. Primary pollutants are substances directly emitted from a source of air pollution into the air. Examples of the primary pollutants include exhaust from combustion of coal and oil, dust from cement plants, sulfur oxides from vehicle exhaust, and the like.

Secondary pollutants are substances produced when the primary pollutants react in the atmosphere to form other new harmful chemicals. Examples of the secondary pollutants include an oxidizing agent which is produced by photochemical reactions of hydrocarbons and nitrogen oxides emitted from automobiles and plants in the presence of sunlight.

Sulfur dioxide is one of a group of gases called sulfur oxides. It is a non-flammable gas which is soluble in water easily and colorless and has a pungent and irritating odor. It is found naturally in the environment and emitted from volcanoes, hot springs, and the like, and reacts with hydrogen sulfide to form sulfur. It is anthropogenically released into the atmosphere when fossil fuels such as coal or oil containing sulfur are burned and the main sources thereof are power plants, heating equipment, metallurgical plants, oil refineries, and various industrial processes.

Carbon monoxide is a colorless, odorless, toxic gas that is produced by incomplete combustion of carbon-containing fuels. A major source of carbon monoxide is automobiles and other sources of carbon monoxide include fuel combustion from plants, a natural source such as forest fires, and an indoor source such as kitchens, cigarette smoke, and district heating.

Nitrogen dioxide is a reddish-brown and highly reactive gas that is produced by oxidation of nitrogen monoxide in the atmosphere, and acts as a precursor to generate ozone by reacting with volatile organic compounds in the atmosphere. A major source of nitrogen dioxide is internal combustion vehicles, high temperature combustion processes, chemical manufacturing processes, and the like, and other sources of nitrogen dioxide include a natural source such as bacteria in the soil.

Ozone is a substance produced by photochemical reactions of hydrocarbons and NOx, volatile organic compounds, and the like emitted from automobiles in the presence of sunlight, and belongs to secondary pollutants. The volatile organic compounds, which are precursors, are produced from a variety of sources, including industrial facilities such as automobiles, chemical plants, and refineries and a natural source.

At present, 61 kinds of substances such as carbon monoxide, ammonia, nitrogen oxides, sulfur oxides, and the like are designated as air pollutants. Among air pollutants, substances that may directly or indirectly cause harm to human health, property, and plant and animal growth are designated as specific pollutants. Currently, 35 kinds of substances such as dioxins, benzene, carbon tetrachloride, formaldehyde, and the like are designated as specific pollutants.

In addition, yellow dust, sand storm, pollen, bacteria in the air, forest fires, and volcanic eruptions are also major causes of air pollution and are a threat to human health. Beijing, China, and Sumatra, Indonesia, have no visibility for more than 30 days a year because of excessive consumption of fossil fuels and artificial fires for palm oil production, causing damage to cities in neighboring countries. In many cities in Southeast Asia, walking through streets without wearing a mask is impossible due to exhaust from motorcycles and automobiles.

Moreover, air pollution is more serious in construction sites, cleaning sites, special manufacturing plants, painting plants, hospitals, and the like. In order to avoid such polluted air, a mask is worn on a face of a person during industrial activities or sports activities. Furthermore, fires of buildings and facilities caused by negligence, earthquakes, and the like, fires of automobiles and trains in tunnels, and toxic gases from agriculture and production sites may immediately take human life.

In recent years, various attempts have been made to protect human respiratory health in such an environment, and health mask development can be cited as an example. Because the main respiratory organs in the human body are the nose and the mouth, a mask is used to protect the nose and the mouth to prevent direct inhaling of air pollutants.

In developing such a mask, the most important thing to emphasize is airtightness. In other words, the development must be aimed at improving technologies to prevent external harmful substances such as fine dust, harmful gas, and the like from entering the mask.

However, a mask with improved airtightness may have high respiration resistance. Accordingly, in the case of a commercially available mask, it may be difficult to respire comfortably with the mask on. Particularly, in the case of a mask made of a cotton material, it may be more difficult to respire with the mask on when moisture from respiration builds up inside the mask and the mask becomes wet with moisture and stick to the nose or mouth.

Thus, there is a demand for a mask capable of enabling efficient respiration of a user while improving airtightness.

With regard to the prior art attention is drawn to US 4 011 865 A from which a pair of small-sized air blowers or air blowing means is known, one being driven by means of an electric motor and the other being manually operated, are respectively connected to one end of two air inlet pipes each of which are selectively switched into operation by means of a three-way valve for providing flow and open feeding of air under pressure into a face covering type mask. An air inlet filter is mounted, as may be required, on the entrance side of the three-way valve. At least, one viewing window is provided on the front surface of the mask body. A transparent guard glass (and a colored, transparent light shielding glass for welding, as may be required) with suitable clearances for allowing the blowing-off of air therearound are oppositely fitted inside a viewing window. In addition, numerous small clearances are formed circumferentially between the inner side peripheral edge of the mask body and the face of a user to provide for air leak-off.

Further, from EP 0 225 744 A1 a powered respirator of self-contained form is known, said power respirator is for use in oxygen-sufficient atmospheres and comprises a visored helmet defining in use a passageway extending from a rear opening across the user's head and face, an electric fan and filter located adjacent the opening to pass respiration air through the passageway, a fan battery housed forwardly of the helmet, and an exhaust valve mounted in the passageway near the user's mouth, the valve operating to open in response to gas pressure similar to that of normal exhalation and including a spring closure mechanism having a decreasing spring rate during opening.

Furthermore, from US 4 258 710 A a respirator of the mask-type is known, said respirator covering the nose and mouth of a wearer to protect him from environments containing air-borne obnoxious particles and gases having a combination of a compressed air inlet regulator on the face of the mask, internal baffling which directs inlet air downwardly past the nose of the wearer, and spaces at the mask's sides in the wearer's cheek area and below his mouth to effect an air flow through and out of the mask in a downward direction with little annoyance to the wearer, to assist in exhausting exhaled breath from the mask, and to form an "air-curtain" at the mask's outlet spaces that prevents entry into the mask of obnoxious particles and gases. The mask is further adapted for quick coupling and uncoupling to and from supply lines of air under pressure and for use with air supply lines of widely varying supply pressures.

### Disclosure

### Technical Problem

Accordingly, the present invention has been made keeping in mind the above problems occurring in the related art, and an objective of the present invention is to provide a mask configured to form an air curtain around a user's chin to block entry of external harmful substances, thereby protecting user's respiratory health.

### Technical Solution

In order to accomplish the above objective, the present invention provides a health mask, including: a body part resting on and covering a partial area of a face, including a respiratory system of a user, the body part having an internal space formed therein; a tight fitting part positioned along a circumference of the body part such that the body part is tightly fitted to the partial area of the face; an ear loop part positioned at opposite sides of the body part such that the body part is secured to the partial area of the face; a filter part positioned at the opposite sides of the body part such that external air is purified and supplied to the internal space of the body part; and an air curtain-forming means provided on the body part such that an air curtain is formed around the body part to block entry of external substances.

Furthermore, in an embodiment of the present invention, the air curtain-forming means include: an air blowing member provided on the body part at a position between the filter part and the ear loop part; a first air passage positioned in the internal space of the body part to allow the air blowing member and a peripheral portion of a user's chin to communicate with each other, such that filtered air is supplied to the peripheral portion of the user's chin; and multiple nozzles communicating with the first air passage and formed at a lower edge of the body part, such that the air curtain is formed along skeletal contours of the user's chin to block the entry of the external substances.

Furthermore, in an embodiment of the present invention, the air curtain-forming means further include: a second air passage positioned in the internal space of the body part to allow the air blowing member and a peripheral portion of a user's nose to communicate with each other, such that the filtered air is supplied to the peripheral portion of the user's nose.

Furthermore, in an embodiment of the present invention, the first air passage is greater than the second air passage in diameter.

Furthermore, in an embodiment of the present invention, the air blowing member includes : a fan seat provided on the body part at the position between the filter part and the ear loop part; a blowing fan positioned outside the fan seat and supplying the filtered air flowing from the filter part to the internal space of the body part; and a battery unit positioned inside the fan seat and connected to the blowing fan, the battery unit being provided to supply electric power to the blowing fan.

Furthermore, in an embodiment of the present invention, the air blowing member further includes: a sound insulation unit positioned on the body part at a position between the fan seat and the ear loop part for sound insulation of the blowing fan.

Furthermore, in an embodiment of the present invention, there is further included a medium member positioned on the body part at a position between the filter part and the blowing fan, such that a medium containing an aroma or drug is contained in the filtered air.

Preferably, a UV coating may be applied to an outer surface of the body part.

Preferably, an anti-fog coating may be applied to an inner surface of the body part.

Preferably, the body part may be formed by nano-injection molding, and a portion thereof on which the air blowing member is mounted is made of relatively more rigid silicone or plastic than a portion thereof that is tightly fitted to the user's skin.

Furthermore, in an embodiment of the present invention, the tight fitting part is provided with an anti-loosening unit positioned at a portion of the tight fitting part that is tightly fitted to a bridge of a nose, such that the body part is prevented from loosening downward along contours of the bridge of the nose, wherein the anti-loosening unit is made of nanofibers obtained by producing multiple fine bristles by injection molding.

Furthermore, in an embodiment of the present invention, the multiple fine bristles is may be inclined upward and arranged in multiple stages in a vertical direction.

Furthermore, in an embodiment of the present invention, the tight fitting part is made of a shape memory resin that contracts according to a user's body temperature and is deformed to conform to skeletal contours of the user's face.

Furthermore, in an embodiment of the present invention, there is further included a temperature adjusting unit cooperating with the filter part such that the air flowing to the internal space of the body part is adjusted in temperature, wherein the temperature adjusting unit includes : a temperature measuring sensor positioned at a center of the body part; and a heating unit positioned on the body part at a position between the filter part and the blowing fan such that the air flowing from the filter part is heated, the heating unit being connected with the battery unit.

Preferably, there may be further included: an environment measuring module measuring an internal space environment of the body part; a danger signal module giving a signal to the user when an external danger occurs; a data processing unit receiving and processing signals from the environment measuring module and the danger signal module; a database unit cooperating with the data processing unit and recording the internal space environment of the body part in real time; a display unit cooperating with the data processing unit and allowing an internal space environment state of the body part to be displayed thereon, an application unit cooperating with the data processing unit and installed on an external smart device, the application unit providing an interface to the user; and a blowing control unit cooperating with the data processing unit and controlling the air blowing member.

Preferably, the environment measuring module may include: a temperature measuring unit cooperating with the temperature measuring sensor and measuring an internal temperature of the body part; a gas measuring unit cooperating with a gas detecting sensor positioned inside the body part and detecting at least one of an oxygen amount, a carbon dioxide amount, and a harmful gas concentration in the internal space of the body part; and a fine dust measuring unit cooperating with a fine dust measuring sensor positioned inside the body part and measuring fine dust concentration in the internal space of the body part.

Preferably, the data processing unit may measure movement speed of the user in cooperation with GPS, and compare an oxygen and carbon dioxide respiration amount for each state of stop, walking, and running states of the user with a real time respiration amount received from the gas measuring unit to check a respiratory health state of the user.

Preferably, the data processing unit may compare the harmful gas concentration detected by the gas measuring unit or the fine dust concentration detected by the fine dust measuring unit with a preset allowable harmful gas concentration or a preset allowable fine dust concentration to check whether replacement of the filter part is required.

Preferably, the danger signal module may include: a sound selection unit cooperating with a sound sensing sensor positioned outside the ear loop part and selecting a preset specific signal; and a vibration signal unit cooperating with a vibration sensor positioned inside the ear loop part and giving a vibration signal to the user in response to the signal from the sound selection unit.

Furthermore, in an embodiment of the present invention, there is further included a goggle part assembled with ear loop parts and the body part such that user's eyes are protected.

Furthermore, in an embodiment of the present invention, there is further included: an image capturing unit positioned on the ear loop part at a position abutting a side of the goggle part such that atmospheric environment or a user's work environment is captured; and a heat sensor positioned on the ear loop part at the position abutting the side of the goggle part such that a living creature in a user's surrounding area is sensed.

Furthermore, in an embodiment of the present invention, there is further included: an image capturing unit positioned on the ear loop part at a position abutting a side of the goggle part such that atmospheric environment or a user's work environment is captured; and a lighting unit positioned on the ear loop part at the position abutting the side of the goggle part such that user's view in a user's surrounding area is secured.

Preferably, there may be further included a microphone unit positioned on the body part at a position corresponding to a peripheral portion of a user's mouth such that a voice signal of the user is transmitted.

Preferably, there may be further included a radioactive concentration measuring sensor positioned on the body part and measuring radioactive concentration of a surrounding environment of the user.

Preferably, there may be further included a sound source output portion positioned at the ear loop part and transmitting information of the internal space environment of the body part or of the surrounding environment of the user in a voice form.

Preferably, the filter part may include: a filter attachment/detachment portion positioned at a side of the opposite sides of the body part; a multi-filter positioned at the filter attachment/detachment portion and comprised of multiple filter layers; and a filter block coupled with the multi-filter and attached to and detached from the filter attachment/detachment portion.

Preferably, the filter attachment/detachment portion may have a magnetic component in powder form that is applied thereto, and the filter block is made of a metal material.

Preferably, the multi-filter may include: a first filter coupled to a filter support and provided in a mesh shape, the first filter having a pore size in a range of 0.02 to 59 µm; and a second filter coupled with the first filter and having a pore size in a range of 0.01 to 59 µm, the second filter removing (ultra) fine dust.

Preferably, the multi-filter may further include a third filter coupled with the second filter and having a pore size in a range of 0.01 to 59 µm, the third filter removing the (ultra) fine dust and containing a deodorizing component.

Preferably, the multi-filter may further include: a fourth filter coupled with the third filter and having a pore size in a range of 0.02 to 59 µm, the fourth filter containing an adsorbent material for removing toxic or harmful substances.

Preferably, the multi-filter may further include: a fifth filter coupled with the fourth filter and having a pore size in a range of 0.01 to 1 mm, the fifth filter containing an aroma component or a therapeutic component for a patient suffering from bronchial disease.

Preferably, pores of respective filters constituting the multi-filter may be arranged alternately in a staggered arrangement.

Preferably, pores of respective filters may be formed using femtosecond lasers.

Preferably, pores of respective filters constituting the multi-filter may be arranged in different patterns.

Preferably, the ear loop part may include: a strip connected to a side of the opposite sides of the body part and looped around the user's ear; and a length adjusting unit positioned at the side of the body part and connected with the strip, the length adjusting unit being provided to adjust a length of the strip.

Preferably, the length adjusting unit may include: a bending body securely positioned at the side of the body part; a bending cover hingedly connected with the bending body; and a bending protrusion positioned on the bending body and provided to adjust the length of the strip by allowing the strip to be wound thereon.

Furthermore, in an embodiment of the present invention, a biomarker unit is positioned on an inner surface of the body part or on the filter part.

### Advantageous Effects

According to the present invention, an air curtain is formed around the user's chin to prevent entry of external harmful substances. In other words, due to the formation of the air curtain, a function of blocking the external harmful substances is exerted along the contours of the user's chin so that airflow along an outer surface of the mask effectively occurs by the Coand 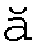 effect and the vortex effect. As a result, a user's exhaled carbon dioxide and external harmful substances are swept downward together with the air curtain to form a fluid barrier, thereby blocking entry of unfiltered substances into the mask.

Furthermore, the air passage is additionally formed at a position corresponding to the user's nose to effectively transfer filtered air, and to appropriately cope with a situation where rapid oxygen consumption is required by a user when running, for example.

Furthermore, a user can adjust speed of the blowing fan by using applications on a smart device, so that when high oxygen consumption is required or concentration of the external harmful substances is high, it is possible to increase the amount of air to be filtered and supplied by increasing the amount of blowing air. This results in positive effects on optimizing a respiratory state of a user.

Furthermore, the medium member may be additionally mounted, so that when a user wants an aroma or to wants to have a specific drug enter the respiratory organs for medical treatment, this can be achieved through the medium member.

Furthermore, the outer surface of the mask has the UV coating, so that it is possible to prevent skin diseases caused by ultraviolet rays in regions, such as Africa, Central and South America, East Asia, and the like, with intense ultraviolet rays.

Furthermore, the inner surface of the mask has the anti-fog coating, so that it is possible to prevent the interior of the mask from fogging when a user exhales, and to prevent occurrence of fogging due to a sudden change in temperature and humidity when a user enters the warm building from a cold outdoors in winter.

Furthermore, the mask has a portion being in contact with the bridge of the nose, the portion having the nano-injection molded fine bristles having a size of 3 to 5 µm attached thereto, so that it is possible to prevent the mask from loosening downward even when a user performs exercise such as running.

Furthermore, the body part of the mask is formed to have elasticity so as to be closed inward, so that it is easy to store the mask when not in use, and the body part of the mask is tightly fitted to the face along the contours of the user's cheeks, chin, and the like due to elasticity when in use, thereby improving blocking ability against external fine dust and harmful gas.

Furthermore, the filter composed of nanofibers having a pore size of approximately 0.1 µm is arranged at the opposite sides of the mask so as to simultaneously block fine dust and viruses, thereby enabling efficient respiration of a user while blocking external fine dust and harmful gas.

Furthermore, the mask of the present invention is provided with the danger signal module cooperating with a smart device, so that when it is difficult for a user to secure his or her view due to severe yellow dust or the like with the mask on, or when a user uses earphones or headphones with the mask on, a sound having a specific waveform such as a police siren sound is detected and then transmitted to a user in a vibration form. Accordingly, the user is notified of external danger and thus can cope therewith.

The mask is provided with the environmental measuring module cooperating with a smart device, such as the oxygen amount and carbon dioxide amount measuring sensor, the harmful gas and fine dust concentration measuring sensor, and the like, so that it is possible to indirectly check a respiratory health state of a user by comparing with a preset respiration amount according to an activity state of the user such as stopping, walking, and running, and to ascertain replacement time of the filter.

Furthermore, the mask is equipped with the temperature adjusting module, which cooperates with a smart device, at a position abutting the filter, so that inhaled air can be heated to a temperature close to the user's body temperature for supply to a user who has weak immunity during the winter season when it is cold.

The mask can also be manufactured in the form of a transparent mask that protects the entire face. Such a front transparent mask is provided with various modules such as a camera, an environment measuring module, a light module, and the like to enable user's convenience.

Furthermore, the mask has the goggles attached thereto and detached therefrom, thereby protecting the user's eyes from various viruses, fine dust, and the like, and the goggles are made of a transparent material and thus can secure user's view.

Furthermore, the image capturing unit, the lighting unit, and the heat sensor are provided in an attachable and detachable manner, so that when firefighters, emergency rescue workers, and the like handle extreme work, it is possible to record a work process and to secure user's view. Even when user's view cannot be secured, the mask can perform additional functions to detect and rescue the human body by using an infrared device.

Furthermore, the display device on which various states of the mask are displayed and notified to a user is equipped on the goggle part, a user can instantly ascertain a state of the mask and quickly cope with the corresponding state.

As a result, the lower edge of the mask uses the air curtain to block entry of fine dust, harmful gas, virus, and the like while permitting free movement of the user's chin, so that a user can communicate clearly with others and can eat food even with the mask on while effectively protecting the respiratory health. Additionally, various sensors and devices that cooperate with a smart device are used to perform a user's respiratory health state check, a filter replacement time notification, temperature adjustment, and the like, so that it is possible to achieve convenience for a user with weak immunity.

### Description of Drawings

FIG. 1 is a front view showing a state in which a first embodiment of a health mask according to the present invention is worn.
FIG. 2 is a side view showing the state in which the first embodiment of the health mask according to the present invention is worn.
FIG. 3 is a side cross-sectional view showing the first embodiment of the health mask of the present invention.
FIG. 4 is a view showing an effect of an air curtain in the invention shown in FIG. 3.
FIG. 5 is a view showing a mounting structure of a filter part and a medium member in the invention shown in FIG. 3.
FIG 6 is a view showing an anti-loosening structure in the invention shown in FIG. 3.
FIG. 7 is a view showing a tight fitting structure in the invention shown in FIG. 3.
FIG. 8 is a view showing a coating structure in the invention shown in FIG. 3.
FIG. 9 is a side cross-sectional view showing a second embodiment of a health mask according to the present invention.
FIG. 10 is a view showing a danger signal notification state of the invention shown in FIG. 9.
FIG. 11 is a view showing a mounting structure of a filter, heat wires, and a medium.
FIG. 12 is a control diagram applied to the invention shown in FIG. 9.
FIG. 13 is a flowchart showing temperature adjustment according to the control diagram shown in FIG. 9.
FIG. 14 is a flowchart showing a filter state check according to the control diagram shown in FIG. 9.
FIG. 15 is a flowchart showing a user's respiratory state check according to the control diagram shown in FIG. 9.
FIG. 16 is a flowchart showing a danger signal notification according to the control diagram shown in FIG. 9.
FIG. 17 is a front view showing a health mask with a goggle part mounted according to the present invention.
FIG. 18 is a side view showing the invention shown in FIG. 17.
FIG. 19 is a view showing an example of a pattern formed in a multi-filter in the present invention.
FIG. 20 is a view showing another example of the pattern formed in the multi-filter in the present invention.
FIG. 21 is a view showing still another example of the pattern formed in the multi-filter in the present invention.
FIG. 22 is a view showing a method of manufacturing the multi-filter according to the present invention.
FIG. 23 is a view showing another method of manufacturing the multi-filter according to the present invention.
FIG. 24 is a view showing an example of an ear loop part in the present invention.
FIG. 25 is a view showing a method of adjusting a length of a strip in the invention shown in FIG. 27.
FIG. 26 is a view showing another example of the ear loop part in the present invention.
FIG. 27 is a view showing another example of a first air passage and an air nozzle in the invention shown in FIG. 3.
FIG. 28 is a view showing still another example of the first air passage and the air nozzle in the invention shown in FIG. 3.
FIG. 29 is a view showing an irregular arrangement of the air nozzle in the invention shown in FIG. 28.
FIG. 30 is a view showing a regular arrangement of the air nozzle in the inventions shown in FIG. 28.
FIG. 31 is a view showing another example of a filter part in the invention shown in FIG. 3.
FIG. 32 is a view showing another example of an air blowing member and the filter part in the present invention.
FIG. 33 is a view showing a mask in which a body part and the goggle part are integrally manufactured in the present invention.
FIG. 34 is a view showing still another example of the first air passage in the present invention.
FIG. 35 is a view showing another example of a second air passage in the present invention.

### Mode for Invention

Hereinafter, preferred embodiments of a health mask according to the present invention will be described in detail with reference to the accompanying drawings.

### [First embodiment]

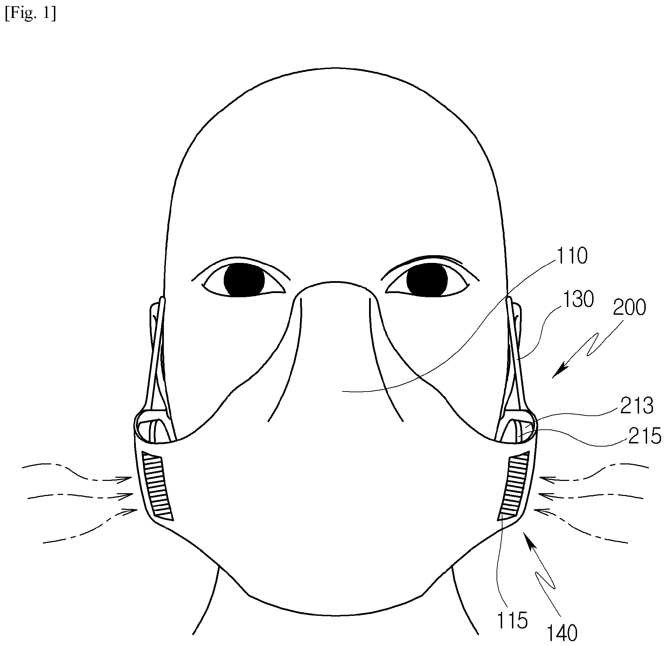

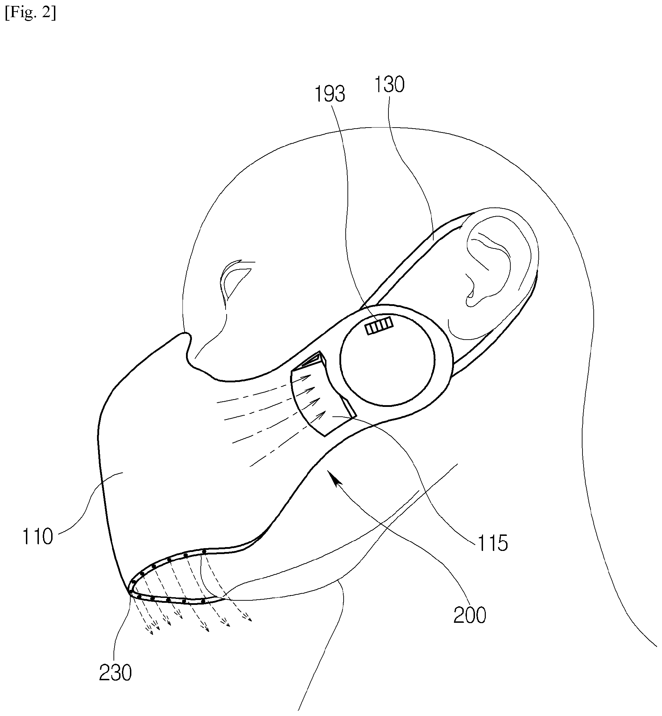

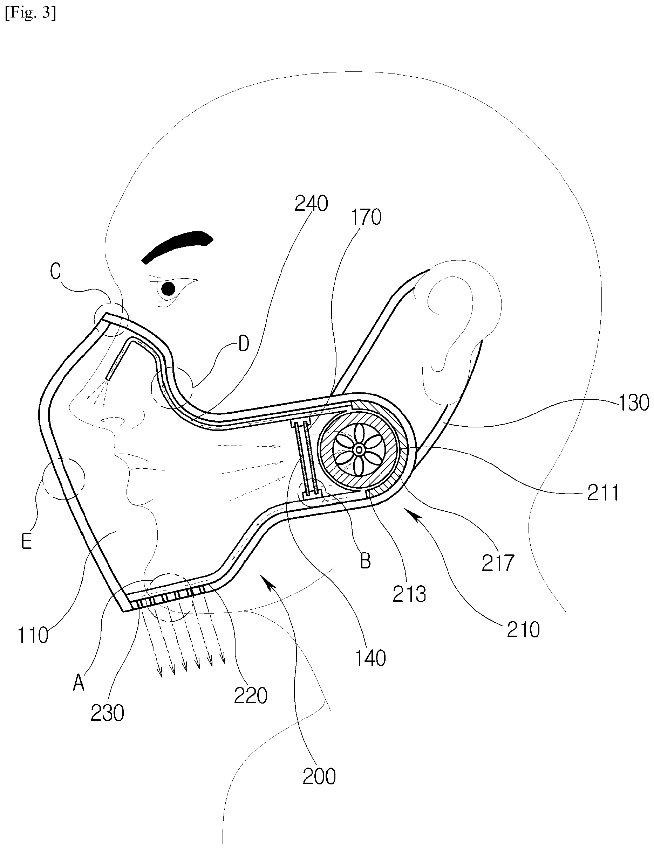

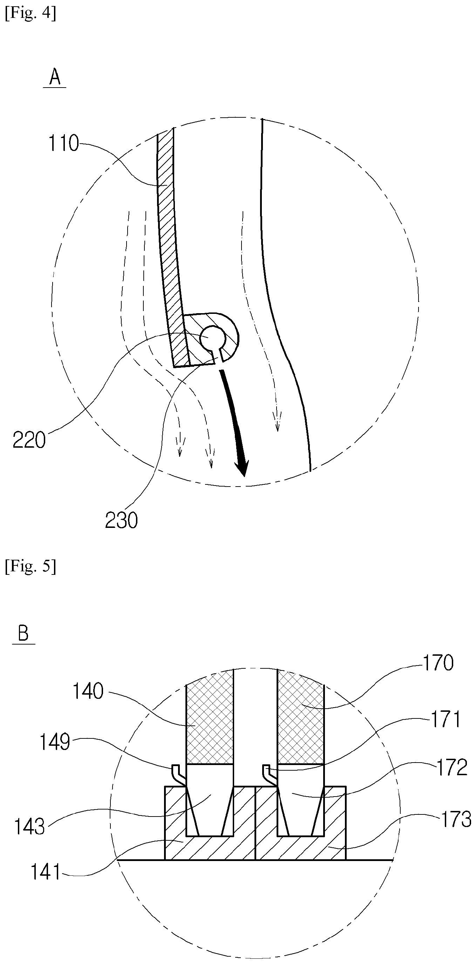

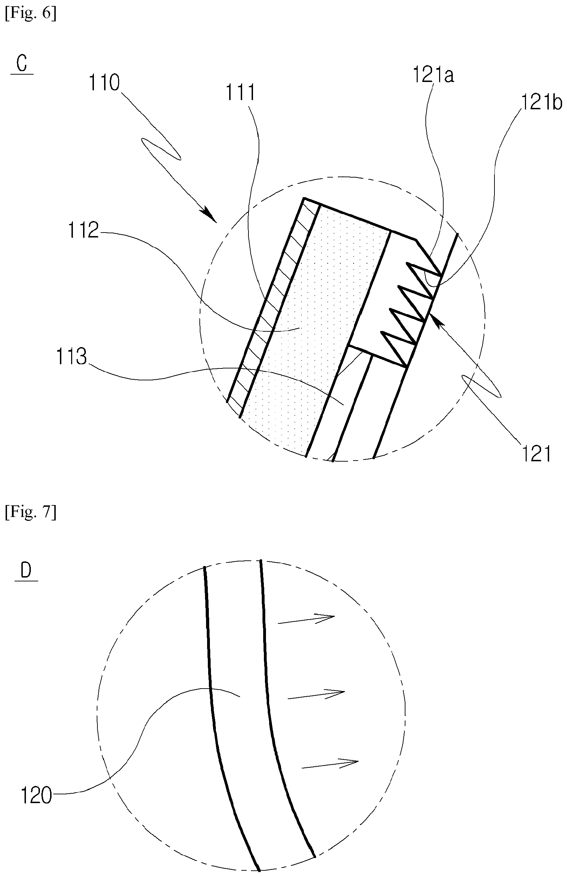

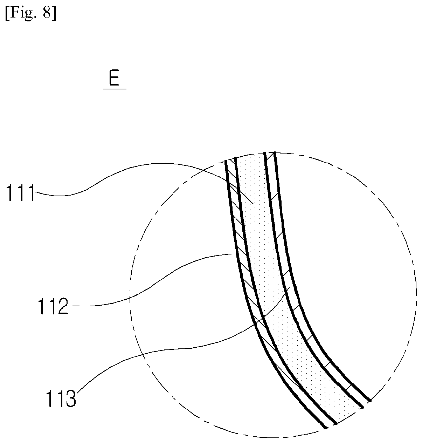

FIG. 1 is a front view showing a state in which a first embodiment of a health mask according to the present invention is worn, FIG. 2 is a side view showing the state in which the first embodiment of the health mask according to the present invention is worn, FIG. 3 is a side cross-sectional view showing the first embodiment of the health mask of the present invention, FIG. 4 is a view showing an effect of an air curtain in the invention shown in FIG. 3, FIG. 5 is a view showing a mounting structure of a filter and a medium the invention shown in FIG. 3, FIG 6 is a view showing an anti-loosening structure in the invention shown in FIG. 3, FIG. 7 is a view showing a tight fitting structure in the invention shown in FIG. 3, and FIG. 8 is a view showing a coating structure in the invention shown in FIG. 3.

Referring to FIGS. 1 to 8, a first embodiment of a health mask according to the present invention includes a body part 110, a tight fitting part 120, an ear loop part 130, a filter part 140, and an air curtain-forming means 200.

First, the body part 110 is rested on and covers a partial area of a face including a respiratory system such as a user's nose, mouth, and the like, and has a predetermined internal space formed therein to provide a space in which respiration take places.

The body part 110 is shaped such that a portion thereof that is in contact with a bridge of a user's nose protrudes upward so as to cover the user's nose when viewed from the front of the face, and a portion thereof that is in contact with user's cheeks is rounded downward in a parabolic shape so as to be tightly fitted thereto in agreement with a facial skeleton of a person. In the embodiment of the present invention, the rounded portion is provided to cover lower portions of cheekbones of a person, but the present invention is not limited thereto, and may be provided in a shape extending upward to a range of covering along the contours of the cheekbones of a person.

The body part 110 may be made of relatively soft silicone or plastic. The selection of such a material is intended to ensure fluidity such that the body part 110 is deformed to conform to the contours of the user's face to be tightly fitted thereto, and to secure ease of storage when not in use.

However, a portion of the body part 110 where an air blowing member 210 of the air curtain-forming means 200 that will be described later is positioned may be made of relatively rigid silicone or plastic so as to support load of the air blowing member 210. In other words, the body part 110 may have a portion made of a soft material and a portion made of a rigid material.

Herein, an intermediate surface 112 of the body part 110 may be formed by nano-injection molding with a size of approximately 0.8 to 1.2 µm and, more preferably approximately 1 µm. This results a surface with nanostructures that are smaller than water particles in size, so that waterproof and windproof effects can be realized. Furthermore, as a nanostructured surface is obtained, a coating material can be more effectively adsorbed to the inside and outside of the intermediate surface 112 of the body part 110.

Furthermore, as shown in FIGS. 3 and 8, a UV coating may be applied to an outer surface 111 of the body part 110. In this case, it is possible to prevent skin diseases that may be caused to a user who is highly exposed to ultraviolet rays in a region where sunlight is direct, such as Africa, Central and South America, East Asia, and the like. Although not shown in the drawings, the UV coating may also be applied to the inside of the body part 110. This may be appropriately selected depending on use environment.

Furthermore, an anti-fog coating may be applied to an inner surface 113 of the body part 110. In this case, it is possible to prevent the interior of the mask from fogging when a user exhales, and to prevent occurrence of fogging due to sudden change in temperature and humidity when a user enters the building in winter. Although not shown in the drawings, the anti-frost coating may also be applied to the outside of the body part 110. This may be appropriately selected depending on use environment.

Furthermore, although not shown in the drawings, an antistatic coating may be applied to the outer surface 111 and the inner surface 113 of the body part 110. Since the antistatic coating is applied, (ultra) fine dust or the like do not stick to the surface of the mask, and contamination of the inside and outside of the mask due to other charged substances can be prevented.

Furthermore, although not shown in the drawings, opposite sides of the body part 110 may be formed by nano-injection molding and may be provided with elasticity, such that the opposite sides of the body part 110 are closed with respect to a central portion thereof when not in use whereas the opposite sides of the body part 110 covers the partial area of the face to be tightly fitted thereto when in use.

In other words, the opposite sides of the body part 110 may be formed to have elasticity from initial manufacturing so as to be closed with respect to the central portion. In this case, ear loop parts 130 connected to the opposite sides of the body part 110 are closed to the central portion, thereby facilitating ease of storage when not in use.

Additionally, the ear loop parts 130 are opened and looped around the user' ears while being held in user's hands when in use. Thus, it is possible to additionally realize an effect that the body part 110 is tightly fitted to the user's skin due to material resilience of the body part 110.

As another example, although not shown in the drawings, a pair of plate-shaped elastic bodies may be positioned at the opposite sides of the body part 110. Such plate-shaped elastic bodies may be provided in a curved configuration in one direction along the contours of the cheeks of a person.

In this case, elasticity acts in a curved direction, and since the opposite sides of the body part 110 are closed with respect to the central portion for ease of storage when not in use whereas the ear loop parts 130 are opened while being held in the user's hands when in use, resilience of the body part 110 acts in a direction toward the central portion thereof whereby it is possible to realize an effect that the body part is tightly fitted to the user's skin when the ear loop parts 130 are looped around the user's ears.

Next, the tight fitting part 120 may be positioned along a circumference of the body part 110 such that the body part 110 is tightly fitted to the partial area of the face.

The tight fitting part 120 may be made of soft silicone or plastic. Due to softness properties, as shown in FIG. 7, the tight fitting part 120 is deformed to conform to the skeletal contours of the user's face so as to be tightly fitted thereto.

As another example of the present invention, the tight fitting part 120 may be made of a shape memory resin. Such a shape memory resin contracts according to the user's body temperature and is deformed to conform to the skeletal contours of the user's face. Because the temperature of the human body is usually approximately 36.5°C, the shape memory resin used as a material of the tight fitting part 120 may be realized such that it contracts at approximately 36.5°C, and expands to return to an original state at a different temperature.

The tight fitting part 120 may be provided with an anti-loosening unit 121 at a portion of the tight fitting part 120 which is tightly fitted to the bridge of the nose such that the body part 110 is prevented from loosening downward along the contours of the bridge of the nose. The anti-loosening unit 121 may be made of nanofibers obtained by producing multiple fine bristles having a size of 3 to 5 µm by injection molding. This may be approximately a size of a seta on the toe pads of a gecko lizard. The multiple fine bristles may be inclined upward and arranged in multiple stages in a vertical direction.

Referring to enlarged views shown in FIGS. 3 and 6, an upper portion 121a of the anti-loosening unit 121 is inclined upward. Accordingly, because resistance by the anti-loosening unit 121 is small, the body part 110 is worn on the user's face by smoothly sliding upward along the bridge of the nose to be tightly fitted thereto. The lower portion 121b of the anti-loosening unit 121 is formed vertically and thus a predetermined amount of friction is generated against a downward direction in which the body part is loosened, resulting in the body part 110 being prevented from loosening downward.

The anti-loosening unit 121 may be provided not only at a position corresponding to the bridge of the nose but also at a position along the tight fitting part 120, such that tight fitting is enhanced along the contour of the user's upper cheeks.

Next, the ear loop part 130 may be positioned at each of the opposite sides of the body part 110 such that the body part 110 is securely tightly fitted to the partial area of the face. The ear loop part 130 may have an elliptical shape as shown in FIG. 3, or may have a hook shape or other various shapes, though it is not shown in the drawings.

Next, the filter part 140 may be positioned at each of the opposite sides of the body part 110 such that external air is purified to be supplied to the internal space of the body part 110. An air inlet port 115 is formed at the opposite sides of the body part 110 and the filter part 140 is positioned on the air inlet port 115. The filter part 140 may be an air filter made of nanofibers having a pore size of 0.01 to 0.59 µm.

In this case, viruses having a size of equal to or greater than 0.05 µm can be filtered out. Since most of the viruses are within a range of 0.05 to 0.1 µm in size, it is possible to protect the respiratory organs of a user from various viruses.

The filter part 140 may be configured in an attachable and detachable manner, and referring to FIGS. 3 and 5, it can be seen than the filter part 140 is mounted on a filter mounting portion 141 in a force-fitted manner or in a magnetic contact manner. A user can push the filter part 140 from the outside of the body part 110 toward the filter mounting portion 141 so as to be fitted thereto upon filter installation, and can remove the filter part 140 by pulling a replacement handle 149 provided at the filter part 140 outside the body part 110 upon filter replacement.

Herein, the filter mounting portion 141 on which the filter part 140 is mounted may be made of rubber, silicone, plastic or the like to facilitate attachment and detachment of the filter part 140. Alternatively, a filter support 143 may be provided in a tapered shape in which a diameter thereof gradually decreases to an end that is received in the filter mounting portion, such that force fitting of the filter part to the filter mounting portion is facilitated. Various shapes that enable the filter part to be easily attached and detached may also be possible. The filter support 143 may also be made of a soft material such as rubber, silicone, plastic, or the like to facilitate attachment and detachment of the filter part.

Next, the air curtain-forming means 200 may be positioned on the body part 110 to form an air curtain around the body part 110 to thereby block entry of external substances. The air curtain forming means 200 may include an air blowing member 210, a first air passage 220, a second air passage 240, and an air nozzle 230.

Referring to FIG. 3, the air blowing member 210 may be provided on the body part 110 at a position between the filter part 140 and the ear loop part 130. The air blowing member 210 may include a fan seat 211, a blowing fan 213, and a battery unit 215.

The fan seat 211 may be provided on the body part 110 at a position between the filter part 140 and the ear loop part 130. The fan seat 211 may have a shape corresponding to a shape of the blowing fan 213 and may have a circular shape in the embodiment of the present invention.

Furthermore, the blowing fan 213 may be a fan having multiple rotor blades arranged thereon. The blowing fan 213 may be positioned outside the fan seat 211 and may function to cause filtered air flowing from the filter part 140 to be supplied to the internal space of the body part 110.

Next, the battery unit 215 may be positioned inside the fan seat 211 and connected with the blowing fan 213, and may be configured to supply electric power to the blowing fan 213. The battery unit 215 may use a secondary battery for weight reduction and continuous use. Examples of the battery unit may include a lithium ion battery, a lithium polymer battery, and the like. Furthermore, the battery unit 215 may use a wireless charging method that is currently used in a smartphone or the like.

Furthermore, as shown in FIG. 2, a battery state display portion 193 in a display form may be positioned abutting the battery unit 215, such that a user can check a current battery state. The battery state display portion may be configured to enable a user to check the battery state through a smartphone in cooperation with applications on the smartphone and may be configured to issue an alert to a user when a remaining battery power level is low.

The battery unit 215 may function to supply electric power to a temperature measuring sensor 161, a gas detecting sensor 313, and a fine dust measuring sensor 315, which will be described later, in addition to a heating unit, and may use a USB charging method or other methods.

Furthermore, the air blowing member 210 may further include a sound insulation unit 217 provided on the body part 110 at a position between the fan seat 211 and the ear loop part 130 for sound insulation of the blowing fan 213. The low-noise blowing fan 213 can be used, but for a user who is sensitive to minute noise, the sound insulation unit 217 may be positioned below the ear loop part 130 in the embodiment of the present invention. In this case, fan noise propagating toward a user's ear is blocked, allowing a user to comfortably wear the mask.

Next, the first air passage 220 may be positioned to allow the air blowing member 210 and a peripheral portion of a chin or cheeks of a user to communicate with each other in the internal space of the body part 110, such that filtered air is supplied to the peripheral portion of the chin or cheeks of a user. Referring to FIGS. 3 and 4, it can be seen that the first air passage 220 is positioned along a lower line of the internal space of the body part 110. The filtered air through the filter part 140 passes through the blowing fan 213 to flow into the first air passage 220.

Herein, the air nozzle 230 communicates with the first air passage 220 so as to form an air curtain along the contours of the peripheral portion of the chin or cheeks of a user to thereby block entry of external substances, and multiple air nozzles may be provided at a lower edge of the body part 110.

The filtered air having flowed along the first air passage 220 is injected toward a lower portion of the body part 110 through the multiple air nozzles 230, and then flows along the contours of the user' chin to stay attached thereto.

At this time, an air curtain is formed, which causes the Coand 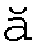 effect. Herein, the Coand 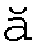 effect is a phenomenon in which a jet flow attaches itself to a nearby surface and remains attached.

Due to the Coand 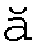 effect, a user's exhaled carbon dioxide and external harmful substances are swept downward together with the air curtain to form a fluid barrier, thereby blocking entry of unfiltered substances into the mask.

Additionally, it can be interpreted that with the Coand 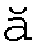 effect, the effect of discharging exhalation and blocking entry of harmful air, which result from the air curtain phenomenon, contributes to effectively discharge the exhalation and the harmful air downward and to effectively form the fluid barrier, whereby entry of the unfiltered substances into the mask is blocked.

Herein, because the lower edge of the body part 110 is a portion that is not tightly fitted to the skin but exerts an effect of being indirectly fitted to the skin using an air curtain, free movement of the user's chin can be permitted. Due to this function, a user can communicate clearly with others even when wearing the mask.

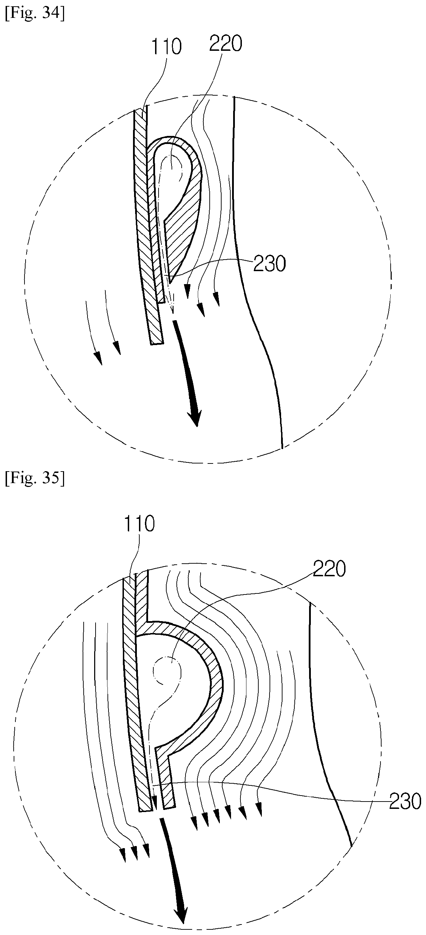

More specific embodiments of the first air passage 220 and the air nozzles 230 for generating the Coand 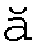 effect are shown in FIGS. 34 and 35.

Next, the second air passage 240 may be positioned to allow the air blowing member 210 and peripheral portions of the user's nose and mouth to communicate with each other in the internal space of the body part 110, such that filtered air is supplied to the peripheral portions of the user's nose and mouth. Although FIG. 3 shows that the second air passage 240 is positioned only at a position corresponding to the peripheral portion of the nose, a separate air passage may extend therefrom to the peripheral portion of the mouth. The second air passage 240 may have an end portion provided with multiple air nozzles, and the air nozzles may have various sizes and shapes depending on application.

Referring to FIG. 3, it can be seen that the second air passage 240 is positioned along an upper line of the internal space of the body part 110. The air filtered through the filter part 140 passes through the blowing fan 213 to flow into the second air passage 240.

Herein, the Vortex effect can be realized. This causes oxygen supplied by the second air passage 240 to swirl in the peripheral portions of the user's nose and mouth to help a user respire. Because a vacuum state can be made in a short time due to the air curtain, this Vortex effect contributes to preventing the case where a user's respiration becomes difficult.

Herein, the first air passage 220 may be greater than the second air passage 240 in diameter. This is to make a difference in the amount of air flowing in each air passage. Accordingly, the amount of filtered air supplied to the first air passage 220 is increased to block entry of external substances, thereby enhancing the effect of the air curtain.

Herein, the second air passage 240 may extend at the peripheral portion of the user's nose to face toward the nose. In this case, the filtered air is directly injected in a direction of nostrils of a user, thereby enabling easier respiration of a user.

Furthermore, a user is allowed to increase or decrease the amount of filtered air supplied to the first and second air passages 220 and 240 by controlling rotational speed of the blowing fan 213 through a blowing control unit 370, which can be arbitrarily determined by a user depending on the degree of air pollution in a surrounding environment of a user wearing the mask.

In the case where the rotational speed of the blowing fan 213 is increased, the amount of air supplied to the user's nose is increased, thereby enabling easier respiration of a user, and the amount of air injected through the air nozzles 230 is increased, thereby significantly enhancing the Coand 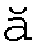 effect due to the air curtain.

Next, in the embodiment of the present invention, there may be further provided a medium member 170 positioned on the body part 110 at a position between the filter part 140 and the blowing part 140, such that a medium, which contains an aroma or a drug for treating asthma, bronchitis, and the like, is contained in the filtered air.

Referring to FIGS. 3 and 5, it can be seen that the medium member 170 is mounted on a medium mounting recess 173 positioned at a rear end side of the filter mounting recess 141. The medium member 170 may be configured in an attachable and detachable manner, and the medium member 170 may be mounted on the medium mounting recess 173 in a force-fitted manner. A user can push the medium member 170 from the outside of the body part 110 toward the medium mounting recess 173 so as to be fitted thereto upon medium installation, and can remove the medium member 170 by pulling a replacement handle 171 from the outside of the body part 110 upon medium replacement, the replacement handle being provided at the filter part.

Herein, the medium mounting recess 173 on which the medium member 170 is mounted may be made of rubber, silicone, plastic or the like to facilitate attachment and detachment of the medium member 170. Alternatively, a medium support 172 may be provided in a tapered shape in which a diameter thereof gradually decreases to an end that is received in the filter mounting portion, such that force fitting of the filter part to the filter mounting portion is facilitated. Various shapes that enable the medium member to be easily attached and detached may also be possible. The medium support 172 may also be made of a soft material such as rubber, silicone, plastic, or the like to facilitate attachment and detachment of the medium member.

As described above, according to the first embodiment of the present invention, the air curtain is used to guide the filtered air in a direction of the user's nose, whereby it is possible to enable efficient respiration of a user and to block external fine dust and harmful gas to thereby provide ultimate protection for user's respiratory health.

### [Second embodiment]

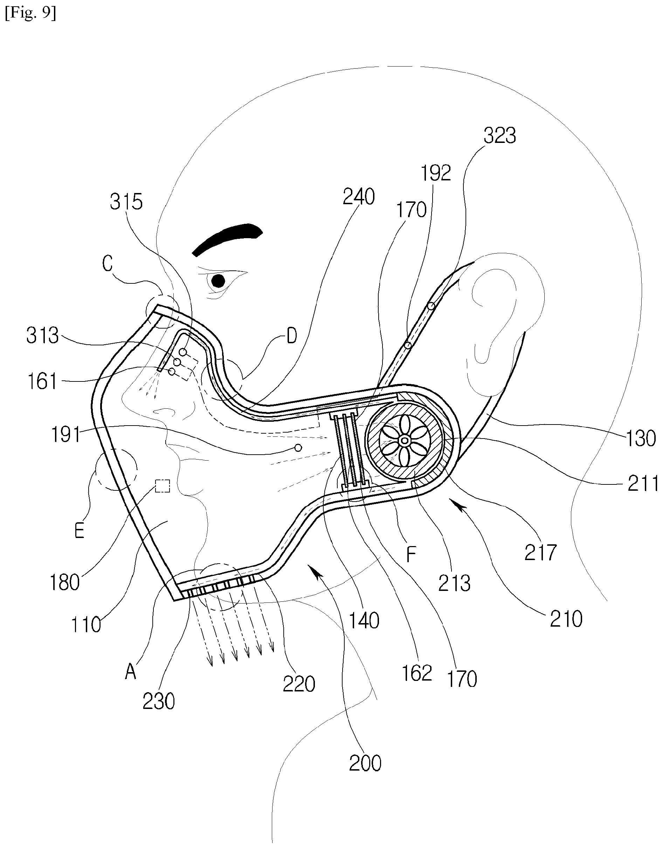

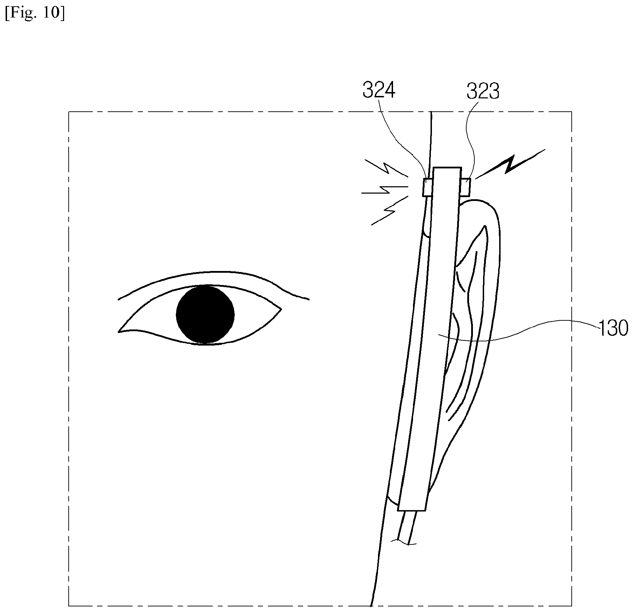

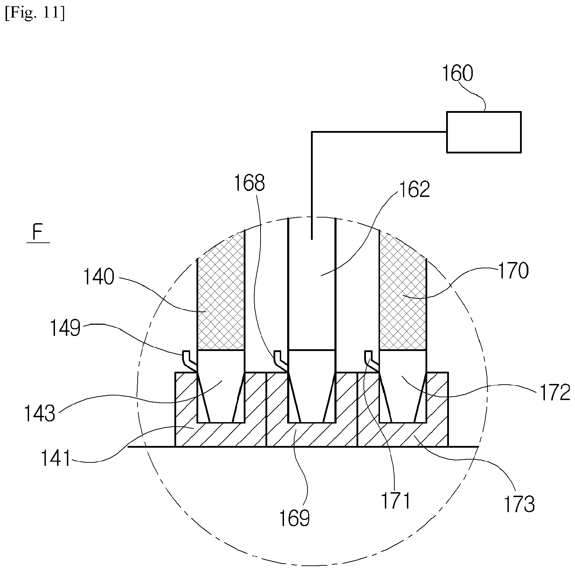

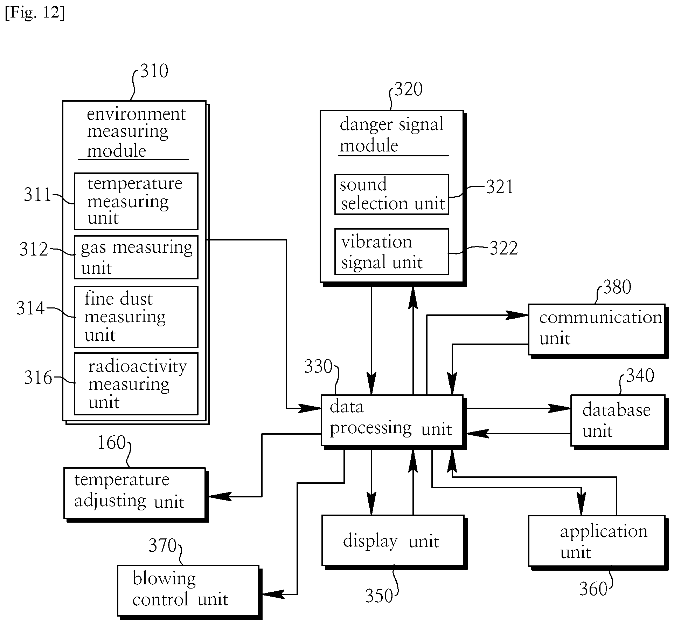

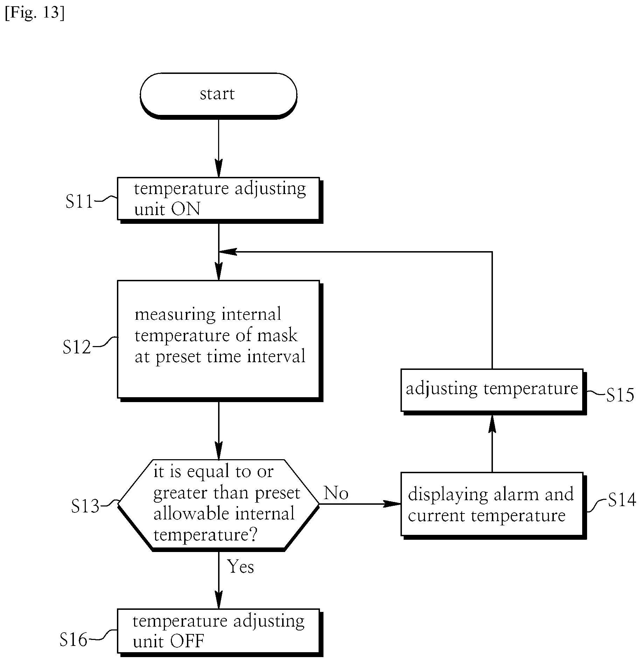

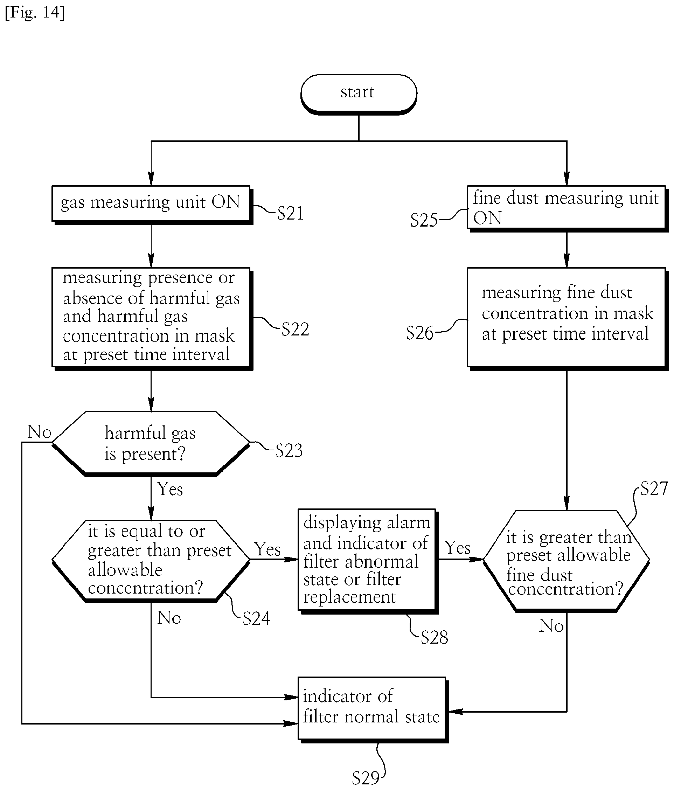

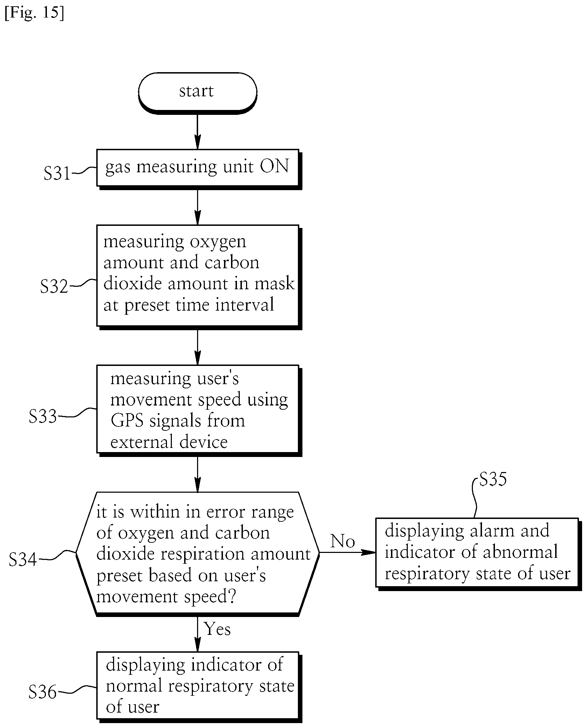

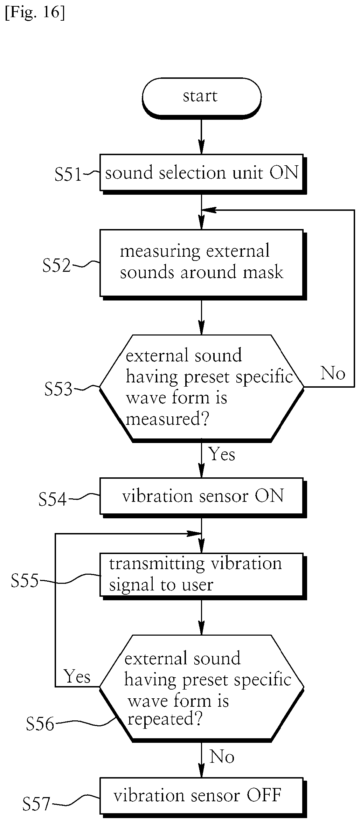

FIG. 9 is a side cross-sectional view showing a second embodiment of a health mask according to the present invention, FIG. 10 is a view showing a danger signal notification state of the invention shown in FIG. 9, FIG. 11 is a view showing a mounting structure of a filter, heat wires, and a medium, FIG. 12 is a control diagram applied to the invention shown in FIG. 9, FIG. 13 is a flowchart showing temperature adjustment according to the control diagram shown in FIG. 9, FIG. 14 is a flowchart showing a filter state check according to the control diagram shown in FIG. 9, FIG. 15 is a flowchart showing a user's respiratory state check according to the control diagram shown in FIG. 9, and FIG. 16 is a flowchart showing a danger signal notification according to the control diagram shown in FIG. 9.

Referring to FIGS.9 to 16, a second embodiment of a health mask according to the present invention includes a body part 110, a tight fitting part 120, an ear loop part 130, a filter part 140, and an air curtain-forming means 200. A basic description of the body part 110, the tight fitting part 120, the ear loop part 130, the filter part 140, and the air curtain-forming means 200 will be given with reference to FIGS. 1 to 8.

First, the body part 110 is rested on and covers a partial area of a face including a respiratory system such as a user's nose, mouth, and the like, and has a predetermined internal space formed therein to provide a space in which respiration take places.

The body part 110 is shaped such that a portion thereof that is in contact with a bridge of a user's nose protrudes upward so as to cover the user's nose when viewed from the front of the face, and a portion thereof that is in contact with user's cheeks is rounded downward in a parabolic shape so as to be tightly fitted thereto in agreement with a facial skeleton of a person. In the embodiment of the present invention, the rounded portion is provided to cover lower portions of cheekbones of a person, but the present invention is not limited thereto, and may be provided in a shape extending upward to a range of covering along the contours of the cheekbones of a person.

The body part 110 may be made of relatively soft silicone or plastic. The selection of such a material is intended to ensure fluidity such that the body part 110 is deformed to conform to the contours of the user's face to be tightly fitted thereto, and to secure ease of storage when not in use.

However, a portion of the body part 110 where an air blowing member 210 of the air curtain-forming means 200 that will be described later is positioned may be made of relatively rigid silicone or plastic so as to support load of the air blowing member 210. In other words, the body part 110 may have a portion made of a soft material and a portion made of a rigid material.

Herein, an intermediate surface 112 of the body part 110 may be formed by nano-injection molding with a size of approximately 0.8 to 1.2 µm and, more preferably approximately 1 µm. This results a surface with nanostructures that are smaller than water particles in size, so that waterproof and windproof effects can be realized. Furthermore, as a nanostructured surface is obtained, a coating material can be more effectively adsorbed to the inside and outside of the intermediate surface 112 of the body part 110.

Furthermore, a UV coating may be applied to an outer surface 111 of the body part 110. In this case, it is possible to prevent skin diseases that may be caused to a user who is highly exposed to ultraviolet rays in a region where sunlight is direct, such as Africa, Central and South America, East Asia, and the like.

Furthermore, an anti-fog coating may be applied to an inner surface 113 of the body part 110. In this case, it is possible to prevent the interior of the mask from fogging when a user exhales, and to prevent occurrence of fogging due to sudden change in temperature and humidity when a user enters the building in winter.

Furthermore, although not shown in the drawings, opposite sides of the body part 110 may be formed by nano-injection molding and may be provided with elasticity, such that the opposite sides of the body part 110 are closed with respect to a central portion thereof when not in use whereas the opposite sides of the body part 110 covers the partial area of the face to be tightly fitted thereto when in use.

In other words, the opposite sides of the body part 110 may be formed to have elasticity from initial manufacturing so as to be closed with respect to the central portion. In this case, ear loop parts 130 connected to the opposite sides of the body part 110 are closed to the central portion, thereby facilitating ease of storage when not in use.

Additionally, the ear loop parts 130 are opened and looped around the user' ears while being held in user's hands when in use. Thus, it is possible to additionally realize an effect that the body part 110 is tightly fitted to the user's skin due to material resilience of the body part 110.

As another example, although not shown in the drawings, a pair of plate-shaped elastic bodies may be positioned at the opposite sides of the body part 110. Such plate-shaped elastic bodies may be provided in a curved configuration in one direction along the contours of the cheeks of a person.

In this case, elasticity acts in a curved direction, and since the opposite sides of the body part 110 are closed with respect to the central portion for ease of storage when not in use whereas the ear loop parts 130 are opened while being held in the user's hands when in use, resilience of the body part 110 acts in a direction toward the central portion thereof whereby it is possible to realize an effect that the body part is tightly fitted to the user's skin when the ear loop parts 130 are looped around the user's ears.

Next, the tight fitting part 120 may be positioned along a circumference of the body part 110 such that the body part 110 is tightly fitted to the partial area of the face.

The tight fitting part 120 may be made of soft silicone or plastic. Due to softness properties, the tight fitting part 120 is deformed to conform to the skeletal contours of the user's face so as to be tightly fitted thereto.

As another example of the present invention, the tight fitting part 120 may be made of a shape memory resin. Such a shape memory resin contracts according to the user's body temperature and is deformed to conform to the skeletal contours of the user's face. Because the temperature of the human body is usually approximately 36.5°C, the shape memory resin used as a material of the tight fitting part 120 may be realized such that it contracts at approximately 36.5°C, and expands to return to an original state at a different temperature.

The tight fitting part 120 may be provided with an anti-loosening unit 121 at a portion of the tight fitting part 120 which is tightly fitted to the bridge of the nose such that the body part 110 is prevented from loosening downward along the contours of the bridge of the nose. The anti-loosening unit 121 may be made of nanofibers obtained by producing multiple fine bristles having a size of 3 to 5 µm by injection molding. This may be approximately a size of a seta on the toe pads of a gecko lizard. The multiple fine bristles may be inclined upward and arranged in multiple stages in a vertical direction.

Referring to enlarged views shown in FIGS. 6 and 9, an upper portion 121a of the anti-loosening unit 121 is inclined upward. Accordingly, because resistance by the anti-loosening unit 121 is small, the body part 110 is worn on the user's face by smoothly sliding upward along the bridge of the nose to be tightly fitted thereto. The lower portion 121b of the anti-loosening unit 121 is formed vertically and thus a predetermined amount of friction is generated against a downward direction in which the body part is loosened, resulting in the body part 110 being prevented from loosening downward.

Next, the ear loop part 130 may be positioned at each of the opposite sides of the body part 110 such that the body part 110 is securely tightly fitted to the partial area of the face. The ear loop part 130 may have an elliptical shape as shown in FIG. 3, or may have a hook shape or other various shapes, though it is not shown in the drawings.

Next, the filter part 140 may be positioned at each of the opposite sides of the body part 110 such that external air is purified to be supplied to the internal space of the body part 110. The filter part 140 may be an air filter made of nanofibers having a pore size of 0.01 to 0.59 µm.

In this case, viruses having a size of equal to or greater than 0.05 µm can be filtered out. Since most of the viruses are within a range of 0.05 to 0.1 µm in size, it is possible to protect the respiratory organs of a user from various viruses.

The filter part 140 may be configured in an attachable and detachable manner, and referring to FIGS. 5 and 9, it can be seen than the filter part 140 is mounted on a filter mounting portion 141 in a force-fitted manner. A user can push the filter part 140 from the outside of the body part 110 toward the filter mounting portion 141 so as to be fitted thereto upon filter installation, and can remove the filter part 140 by pulling a replacement handle 149 provided at the filter part 140 outside the body part 110 upon filter replacement.

Herein, the filter mounting portion 141 on which the filter part 140 is mounted may be made of rubber, silicone, plastic or the like to facilitate attachment and detachment of the filter part 140. Alternatively, a filter support 143 may be provided in a tapered shape in which a diameter thereof gradually decreases to an end that is received in the filter mounting portion, such that force fitting of the filter part to the filter mounting portion is facilitated. Various shapes that enable the filter part to be easily attached and detached may also be possible. The filter support 143 may also be made of a soft material such as rubber, silicone, plastic, or the like to facilitate attachment and detachment of the filter part.

Next, the air curtain-forming means 200 may be positioned on the body part 110 to form an air curtain around the body part 110 to thereby block entry of external substances. The air curtain forming means 200 may include an air blowing member 210, a first air passage 220, a second air passage 240, and an air nozzle 230.

Referring to FIG. 9, the air blowing member 210 may be provided on the body part 110 at a position between the filter part 140 and the ear loop part 130. The air blowing member 210 may include a fan seat 211, a blowing fan 213, and a battery unit 215.

The fan seat 211 may be provided on the body part 110 at a position between the filter part 140 and the ear loop part 130. The fan seat 211 may have a shape corresponding to a shape of the blowing fan 213 and may have a circular shape in the embodiment of the present invention.

Furthermore, the blowing fan 213 may be a fan having multiple rotor blades arranged thereon. The blowing fan 213 may be positioned outside the fan seat 211 and may function to cause filtered air flowing from the filter part 140 to be supplied to the internal space of the body part 110.

Next, the battery unit 215 may be positioned inside the fan seat 211 and connected with the blowing fan 213, and may be configured to supply electric power to the blowing fan 213. The battery unit 215 may use a secondary battery for weight reduction and continuous use. Examples of the battery unit may include a lithium ion battery, a lithium polymer battery, and the like.

The battery unit 215 may function to supply electric power to a temperature measuring sensor 161, a gas detecting sensor 313, and a fine dust measuring sensor 351, which will be described later, in addition to a heating unit 162, and may use a USB charging method or other methods.

Furthermore, the air blowing member 210 may further include a sound insulation unit 217 provided on the body part 110 at a position between the fan seat 211 and the ear loop part 130 for sound insulation of the blowing fan 213. The low-noise blowing fan 213 can be used, but for a user who is sensitive to minute noise, the sound insulation unit 217 may be positioned below the ear loop part 130 in the embodiment of the present invention. In this case, fan noise propagating toward a user's ear is blocked, allowing a user to comfortably wear the mask.

Next, the first air passage 220 may be positioned to allow the air blowing member 210 and a peripheral portion of a chin of a user to communicate with each other in the internal space of the body part 110, such that filtered air is supplied to the peripheral portion of the chin of a user. Referring to FIGS. 4 and 9, it can be seen that the first air passage 220 is positioned along a lower line of the internal space of the body part 110. The filtered air through the filter part 140 passes through the blowing fan 213 to flow into the first air passage 220.

Herein, the air nozzle 230 communicates with the first air passage 220 so as to form an air curtain along the contours of the peripheral portion of the chin of a user to thereby block entry of external substances, and multiple air nozzles may be provided at the lower edge of the body part 110.

The filtered air having flowed along the first air passage 220 is injected toward a lower portion of the body part 110 through the multiple air nozzles 230, and then flows along the contours of the user' chin to stay attached thereto.

Herein, an air curtain is formed, which causes the Coand 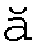 effect. Herein, the Coand 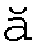 effect is a phenomenon in which a jet flow attaches itself to a nearby surface and remains attached.

Due to the Coand 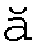 effect, a user's exhaled carbon dioxide and external harmful substances are swept downward together with the air curtain to form a fluid barrier, thereby blocking entry of unfiltered substances into the mask.

Next, the second air passage 240 may be positioned to allow the air blowing member 210 and peripheral portions of the user's nose and mouth to communicate with each other in the internal space of the body part 110, such that filtered air is supplied to the peripheral portion of the user's nose.

Referring to FIG. 9, it can be seen that the second air passage 240 is positioned along an upper line of the internal space of the body part 110. The air filtered through the filter part 140 passes through the blowing fan 213 to flow into the second air passage 240.

Herein, the first air passage 220 may be greater than the second air passage 240 in diameter. This is to make a difference in the amount of air flowing in each air passage. Accordingly, the amount of filtered air supplied to the first air passage 220 is increased to block entry of external substances, thereby enhancing the effect of the air curtain.

Herein, the second air passage 240 may extend at the peripheral portion of the user's nose to face toward the nose. In this case, the filtered air is directly injected in a direction of nostrils of a user, thereby enabling easier respiration of a user.

Furthermore, a user is allowed to increase or decrease the amount of filtered air supplied to the first and second air passages 220 and 240 by controlling rotational speed of the blowing fan 213 through a blowing control unit 370, which can be arbitrarily determined by a user depending on the degree of air pollution in a surrounding environment of a user wearing the mask.

In the case where the rotational speed of the blowing fan 213 is increased, the amount of air supplied to the user's nose is increased, thereby enabling easier respiration of a user, and the amount of air injected through the air nozzles 230 is increased, thereby significantly enhancing the Coand 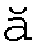 effect due to the air curtain.

Next, in the embodiment of the present invention, there may be further provided a medium member 170 positioned on the body part 110 at a position between the filter part 140 and the blowing part 140, such that a medium, which contains an aroma or a drug, is contained in the filtered air.

Referring to FIGS. 5 and 9, it can be seen that the medium member 170 is mounted on a medium mounting recess 173 positioned at a rear end side of the filter mounting recess 141. The medium member 170 may be configured in an attachable and detachable manner, and the medium member 170 may be mounted on the medium mounting recess 173 in a force-fitted manner. A user can push the medium member 170 from the outside of the body part 110 toward the medium mounting recess 173 so as to be fitted thereto upon medium installation, and can remove the medium member 170 by pulling a replacement handle 171 from the outside of the body part 110 upon medium replacement, the replacement handle being provided at the filter part.

Herein, the medium mounting recess 173 on which the medium member 170 is mounted may be made of rubber, silicone, plastic or the like to facilitate attachment and detachment of the medium member 170. Alternatively, a medium support 172 may be provided in a tapered shape in which a diameter thereof gradually decreases to an end that is received in the filter mounting portion, such that force fitting of the filter part to the filter mounting portion is facilitated. Various shapes that enable the medium member to be easily attached and detached may also be possible. The medium support 172 may also be made of a soft material such as rubber, silicone, plastic, or the like to facilitate attachment and detachment of the medium member.

Next, the second embodiment of the present invention differs from the first embodiment of the present invention in that there is further provided a temperature adjusting unit 160 cooperating with the filter part 140, such that air flowing to the internal space of the body part 110 is adjusted in temperature. The temperature adjusting unit 160 may raise external cold air in temperature such that temperature-raised air is supplied to prevent a decrease in immunity of people in winter or of people who live in cold regions, thereby functioning to mitigate occurrence of respiratory diseases such as the common cold.

The temperature adjusting unit 160 may include a temperature measuring sensor 161 and a heating unit 162.

First, the temperature measuring sensor 161 may be positioned at a center of the body part 110 which is corresponding to the peripheral portion of the nose. Specifically, the temperature measuring sensor is positioned at a position of the body part 110 corresponding to where the user's nose is located, and the temperature of air flowing into the user's nose is measured.

Furthermore, the heating unit 162 may be positioned on the body part at a position between the filter part 140 and the blowing fan 213 such that air flowing through the filter part is heated, and may be connected with the battery unit 215. Referring to FIG. 9, the heating unit 162 is positioned inside the filter part 140 and heats filtered air flowing through the filter part from outside.

More specifically, the heating unit may be positioned between the filter part and the medium member 170. A heating wire mounting recess 169 is provided between the filter mounting recess 141 and the medium mounting recess 173. The heating unit 162 is force-fitted to the heating wire mounting recess 169 and is removed by pulling a replacement handle 168. In this case, the air filtered through the filter part may be heated by the heating unit within a predetermined temperature range and then may pass through the medium member 170 to be a working medium containing an aroma or a drug necessary for a user.

The heating unit 162 may be configured as heat wires arranged in a zigzag arrangement, and a heating temperature may be suitably in a range of approximately 34 to 38°C. Upon operation within the above temperature range, a user does not feel that the filtered air heated is hot because the filtered air heated is in a temperature range similar to the normal human body temperature range. In this case, the filtered air for supply is heated such that the temperature thereof increases by approximately 3 to 4°C, so that it is possible to mitigate to some extent a decrease in immunity due to cold air.

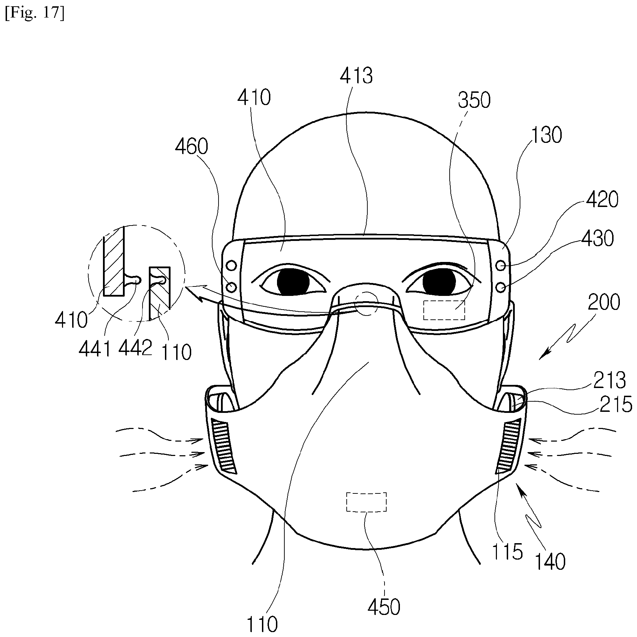

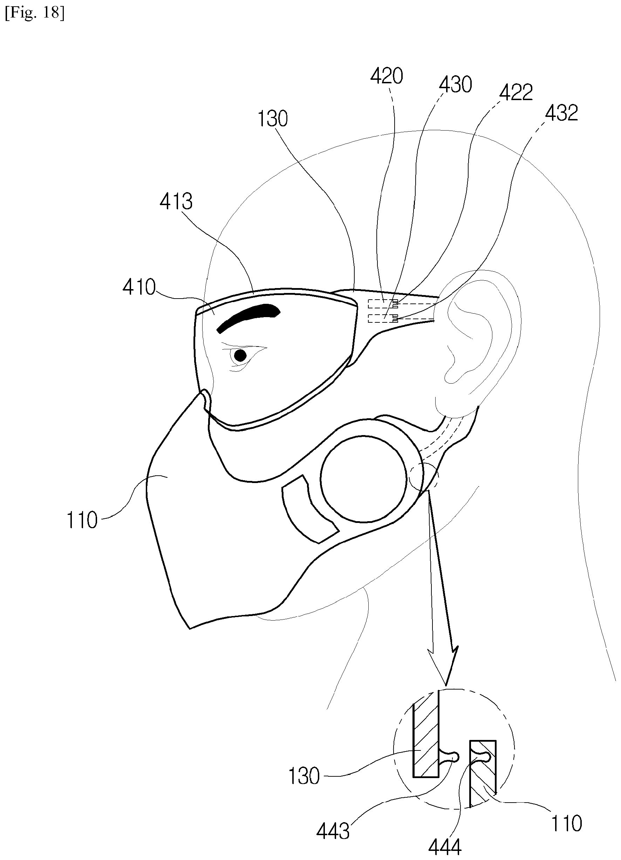

Next, referring to FIGS. 17 and 18, the body part 110 and a goggle part 410 may be integrally manufactured. In this case, a single-integral goggle-type dustproof health mask may be manufactured in which the shape and function inherent to the goggle part 410 are added to the shape and function inherent to the mask.

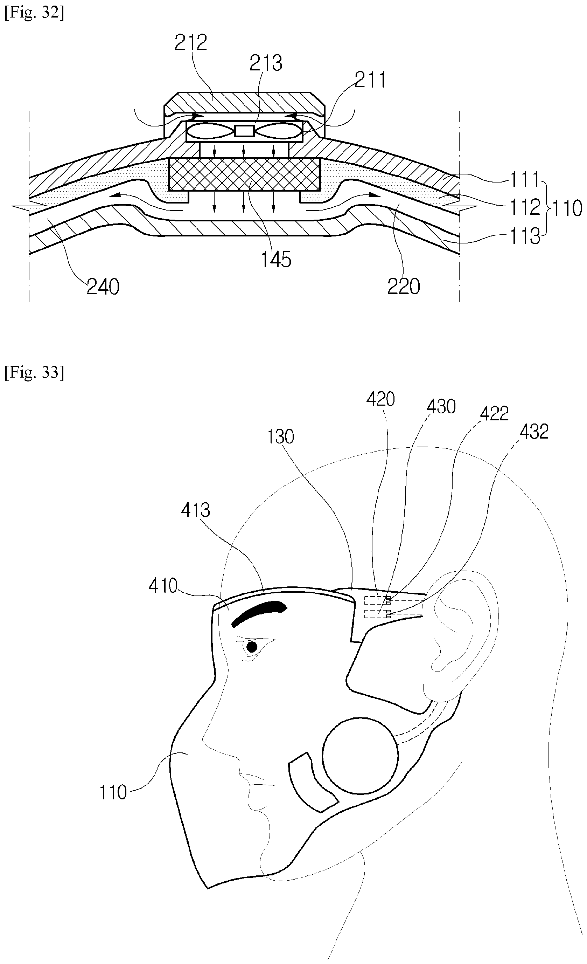

Another example of such an integral mask is shown in FIG. 33.

As another example, the goggle part 410 may be assembled to the body part 110 in an attachable and detachable manner. In this case, the body part 110 or the goggle part 410 can be disassembled from each other upon cleaning, parts replacement, and the like, and additional effects such as ease of washing, a reduction in replacement cost, and the like can be realized.

The goggle part 410 is made of a transparent material to secure user's view and functions to prevent various viruses, fine dust, and the like from penetrating into the user's eyes.

Furthermore, the goggle part 410 may be integrally formed with the ear loop parts 130. Herein, the ear loop parts 130 may be attachable to and detachable from the body part 110. Referring to FIG. 18, the ear loop part 130 has a protrusion 443 formed at an end thereof, and the body part 110 has a recess 444 formed at an end thereof.

A user can insert the protrusion 443 into the recess 444 to assemble the ear loop part 130 to the body part 110 and, conversely, can remove the protrusion 443 from the recess 444 upon mask washing, mask replacement, or the like.

Furthermore, referring to FIG. 17, the goggle part 410 has a protrusion 441 formed at a central portion thereof, and the body part 110 has a recess 442 formed at a center portion thereof. Accordingly, a user can insert the protrusion 441 into the recess 442 to assemble the goggle part 410 to the body part 110 and, conversely, can remove the protrusion from the recess upon disassembling.

As still another example, the goggle part 410 or the ear loop parts 130 may be attached to the body part 110 in a magnetic contact manner.

The goggle part 410 has a goggle tight fitting portion 410 positioned along a circumference thereof. Accordingly, when a user wears the goggle part 410, the goggle tight fitting portion conforms to the skeletal contours of the cheekbones and eyebrows of a user so as to be tightly fitted thereto. The goggle tight fitting portion 413 may be made of a material the same as that of the tight fitting part 410.

The goggle part 410 may have a display unit 350 positioned thereon, which will be described below. A user can check various states of the mask through the display unit 350 and quickly cope with the corresponding states. The display unit 350 may be a display such as an LCD, an LED, a Full HD, a QHD, or the like.

The goggle part 410 may have an image capturing unit 420, a lighting unit 460, a heat sensor 430, and the like that are positioned at opposite sides thereof. When firefighters, emergency rescue workers, and the like handle extreme work, the image capturing unit 420 allows recording and monitoring a work situation.

The lighting unit 460 performs a function of securing user's view in a dark work environment. The heat sensor 430 may be an infrared device or the like, and may allow detecting a person using infrared rays and enhancing performance of rescue work when user's view is difficult to secure.

As shown in FIG. 18, the image capturing unit 420, the lighting unit 460, the heat sensor 430 and the like may be selectively attached to and detached from a first jack portion 422 and a second jack portion 432. In the embodiment of the present invention, the number of connection jacks is two, but is not limited thereto, and the number may be appropriately changed according to a work environment. The first jack portion 422 and the second jack portion 432 may be connected with the battery unit.

The battery unit 215 may be positioned inside the fan seat 211 and connected with the blowing fan 213, and may be provided to supply electric power to the blowing fan 213. The battery unit 215 may use a secondary battery for weight reduction and continuous use. Examples of the battery unit may include a lithium ion battery, a lithium polymer battery, and the like. Furthermore, the battery unit 215 may use a wireless charging method that is currently used in a smartphone or the like.

The ear loop parts 130 are attachable to and detachable from the body part 110, and accordingly a user can remove the ear loop parts 130 to recharge the battery unit 215. Herein, the blowing fan 213 can be mounted so as to be removable from the fan seat 211, and accordingly a user can separately remove only the blowing fan 213 to individually wash and clean the body part 110 and the blower fan 213.

Next, a microphone unit 450 may be mounted on the body part 110 at a position corresponding to the peripheral portion of the mouth. The microphone unit 450 performs a function of transmitting a user's voice more clearly in a state of wearing a mask. The microphone unit 450 may have a function of simply increasing the volume of the user's voice like a loudspeaker, and of performing wireless transmission as well whereby a user can transmit atmospheric environment condition or a current state of work to another user or an administrator in real time.

Referring to FIG. 12, the second embodiment of the present invention may include various control programs that can cooperate with a smart device, including an environment measuring module 310, a danger signal module 320, a data processing unit 330, a database unit 340, the display unit 350, a communication unit 380, the temperature adjusting unit 160, and an application unit 360.

The environment measuring module 310 is a module for measuring an internal space environment of the body part 110. The environment measuring module 310 may include a temperature measuring unit 311, a gas measuring unit 312, and a fine dust measuring unit 314.

The temperature measuring unit 311 cooperates with the temperature measuring sensor 161 and performs a function of measuring the internal temperature of the body part 110. Furthermore, the gas measuring unit 312 cooperates with the gas detecting sensor 313 positioned inside the body part 110 and performs a function of detecting at least one of an oxygen amount, a carbon dioxide amount, and a preset harmful gas concentration in the internal space of the body part 110. Furthermore, the fine dust measuring unit 314 cooperates with the fine dust measuring sensor 315 positioned inside the body part 110 and performs a function of measuring fine dust concentration in the internal space of the body part 110.

Referring to FIG. 9, the temperature measuring sensor 161, the gas detecting sensor 313, and the fine dust measuring sensor 315 may be positioned on the body part 110 at positions corresponding to peripheral portions of the user's respiratory organs such as the user's nose, mouth, and the like. Though not necessarily limited thereto, it is possible to provide relatively accurate measurement when positioned at the positions corresponding to the peripheral portions of the user's respiratory organs. Herein, harmful gas concentration or fine dust concentration may be measured in PPM units.

Furthermore, the danger signal module 320 performs a function of giving a signal to a user who moves with the health mask or when an external danger occurs. The danger signal module 320 may include a sound selection unit 321 and a vibration signal unit 322.

Referring to FIG. 10, the sound selection unit 321 may be provided to cooperate with a sound sensing sensor 323 positioned outside the ear loop part 130 and to select a preset specific signal. The vibration signal unit 322 may be provided to cooperate with a vibration sensor 324 positioned inside the ear loop part 130 and to give a vibration signal to a user in response to the signal from the sound selection unit 321.

For example, a user who wears the health mask may not be able to recognize the external danger such as automobiles, trains, and the like in a state where a user is unable to secure a clear view due to severe yellow dust or sand storms or is moving while listening to the sound through earphones or headphones.

At this time, a specific waveform of a horn sound of an automobile, a siren sound of an emergency vehicle, and the like is input to the database unit 340 in advance, and when such a specific waveform is detected, a user is notified of the danger.

The data processing unit 330 receives signals from the environment measuring module 310 and the danger signal module 320 and performs processing corresponding thereto. The database unit 340 cooperates with the data processing unit 330 and performs a function of recording the internal space environment of the body part 110 in real time.

Furthermore, the display unit 350 cooperates with the data processing unit 330 and is installed on an external smart device and is provided to allow an internal space environment state of the body part 110 to be displayed thereon. The application unit 360 cooperates with the data processing unit 330 and is installed on the external smart device and performs a function of providing an interface to a user. The communication unit 380 performs a function of receiving GPS signals.

The display unit 350 and the application unit 360 may be provided together on a single screen on a smart device.

Next, the blowing control unit 370 adjusts rotational speed of the blowing fan 213 to increase or decrease the amount of filtered air supplied to the first and second air passages. This can be arbitrarily determined by a user depending on the degree of air pollution in the surrounding environment of a user wearing the mask.

When air pollution in the surrounding environment of a user is severe, a user increases the rotational speed of the blowing fan 213 to efficiently block entry of external harmful substances. As the amount of air to be filtered increases, the amount of air supplied to the user's nose increases, thereby enabling easier respiration of a user and at the same time the amount of air injected through the air nozzles 230 also increases, leading to an improvement in the Coand 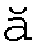 effect due to the air curtain. In other words, it is possible to achieve an improved blocking ability against harmful substances.

Hereinafter, an operation process by the various control programs will be described.

First, referring to FIG. 13, an operation process of the temperature adjusting unit 160 is shown. When a user turns on an application installed on a smart device and presses a temperature adjustment button provided in the application, the temperature adjusting unit 160 is turned on (S11). In this case, the internal temperature of the mask is measured at a preset time interval by the temperature measuring sensor 161. The time interval may be 30 minutes, 1 hour, and the like, which can be arbitrarily selected by a user via the application (S12).

The measured temperature is transmitted to the data processing unit 330, and the data processing unit 330 compares and determines whether the measured temperature is equal to or greater than a preset allowable internal temperature (input to the database unit 340) (S13). When the allowable internal temperature of the mask preset by a user is 15°C and the measured internal temperature of the mask is 12°C, a current internal temperature of the mask is lower than the preset allowable internal temperature in the algorithm, so that an alarm and the current internal temperature of the mask are displayed on the display unit 350 installed on the smart device (S14).

Meanwhile, the temperature adjusting unit 160 operates the heating unit 162 to heat air flowing through the filter part 140 (S15). When the flowing air is heated by approximately 3 to 4°C and then when the temperature measuring sensor 161 measures again the internal temperature of the mask and the measured internal temperature approaches the preset allowable internal temperature of the mask, the temperature adjusting unit 160 is automatically turned off (S16) . A user may also arbitrarily turn off the temperature adjusting unit with the button provided in the application.

Next, referring to FIG. 14, an operation process of the gas measuring unit 312 and the fine dust measuring unit 314 is shown. First, a description of the gas measuring unit 312 is given. When a user turns on the application installed on the smart device and presses a gas measurement button provided in the application, the gas measuring unit 312 is turned on (S21). In this case, presence or absence of harmful gas and harmful gas concentration in the mask are measured at a preset time interval by the gas detecting sensor 313. The time interval may be 6 hours, 12 hours, and the like, which can be arbitrarily selected by a user via the application (S22).

The measured presence or absence of harmful gas and the measured harmful gas concentration are transmitted to the data processing unit 330. When no harmful gas is present, an indicator of a normal filter state is immediately displayed on the display unit 350 installed on the smart device of a user (S23 and S29).

In contrast, when harmful gas is detected, the data processing unit 330 compares and determines whether the harmful gas concentration is equal to or greater than a preset allowable harmful gas concentration (input to the database unit 340) (S23 and S24). For example, when the allowable harmful gas concentration of the mask preset by a user is 50 PPM and the measured harmful gas concentration in the mask is 70 PPM, a current harmful gas concentration in the mask is greater than the preset allowable harmful gas concentration in the algorithm, so that an alarm, the current harmful gas concentration in the mask, and an indicator of filter replacement are displayed on the display unit 350 (S28).

When the measured harmful gas concentration is lower than the preset harmful gas concentration, the indicator of the filter normal state is displayed on the display unit 350, which means that the filter can still be used (S29).

Next, a description of the fine dust measuring unit 314 is given. When a user turns on the application installed on the smart device and presses a fine dust measurement button provided in the application, the fine dust measuring unit 314 is turned on (S25). In this case, fine dust concentration in the mask is measured at a preset time interval by the fine dust measuring sensor 315. The time interval may be 3 hours, 6 hours, and the like, which can be arbitrarily selected by a user via the application (S26).

The measured fine dust concentration is transmitted to the data processing unit 330, and the data processing unit 330 compares and determines whether the measured fine dust concentration is equal to or greater than a preset allowable fine dust concentration (input to the database unit 340) (S27). For example, when the allowable fine dust concentration of the mask preset by a user is 81 PPM and the measured fine dust concentration in the mask is 100 PPM, current fine dust concentration in the mask is greater than the preset allowable fine dust concentration in the algorithm, so that an alarm, the current fine dust concentration in the mask, and the indicator of the filter replacement are displayed on the display unit 350 (S28).

When the measured fine dust concentration is lower than the preset fine dust concentration, the indicator of the filter normal state is displayed on the display unit 350, which means that the filter can still be used (S29).

The allowable fine dust concentration may be set based on the current ambient air quality standards of Korea. For example, referring to a standard of 0 to 30 PPM (good), a standard of 31 to 80 PPM (normal), a standard of 81 to 150 PPM (bad), a standard of equal to or greater than 151 PPM (very bad), a user can set the standard of 81 PPM to the allowable fine dust concentration by using the application unit 360.

Next, referring to FIG. 15, an operation process of the gas measuring unit 312 for measuring the oxygen amount and the carbon dioxide amount is shown. A description of the gas measuring unit 312 is given. When a user turns on the application installed on the smart device and presses a gas measurement button provided in the application, the gas measuring unit 312 is turned on (S31). In this case, the oxygen amount and the carbon dioxide amount respired by a user in the mask are measured at a preset time interval by the gas detecting sensor 313. The time interval may be a respiration amount per minute, a respiration amount per hour, and the like, which can be arbitrarily selected by a user via the application (S32).

Next, the communication unit 380 receives the GPS signals, and a movement distance and a movement time of a user are detected for each time to measure a current movement speed of a user (S33).

The measured movement speed is transmitted to the data processing unit 330 and the data processing unit 330 compares an oxygen and carbon dioxide respiration amount (input to the database unit 340) preset for each state of stop, walking, and running states of a user with a real time respiration amount received from the gas measuring unit 312 to determine whether the real time respiration amount is within an error range of the preset respiration amount (S34). When it is determined that the real time respiration amount does not coincide with the respiration amount preset for each state by a user, an alarm and an indicator of a abnormal respiratory state of a user are displayed on the display unit 350 (S35).

When the measured respiration amount is within the error range of the preset respiratory amount, an indicator of a normal respiratory state of a user is displayed (S36).

Next, referring to FIG. 16, an operation process of the danger signal module 320 is shown. When a user turns on the application installed on the smart device and presses a danger signal notification button provided in the application, the sound selection unit 321 is turned on (S51). In this case, external sounds around the mask are measured in real time by a sound measuring sensor (S52).

Herein, when an external sound having a preset specific waveform (input to the database unit 340) is repeated until measurement take places, when the sound having the preset specific waveform is a horn sound of an automobile, a siren sound of an emergency vehicle, or the like, and when the sound is measured (S53), the vibration sensor 324 is turned on (S54) and transmits a vibration signal to a user (S55). In the second embodiment of the present invention, the vibration sensor 324 is mounted on the ear loop part 130, so that when an external danger signal is detected, a user can sense danger by vibration sensed through the ear.

Thereafter, the sound measuring sensor continuously measures the external sounds, and determines whether the external sound having the preset specific waveform is repeated (S56). When the external sound having the preset specific waveform stops, the vibration sensor 324 is turned off (S57).

As described above, according to the second embodiment of the present invention, functions of measuring a mask internal environment, checking a user's health state, and a danger signal notification through the control programs that can cooperate with the smart device are performed, whereby user's convenience can be further improved.

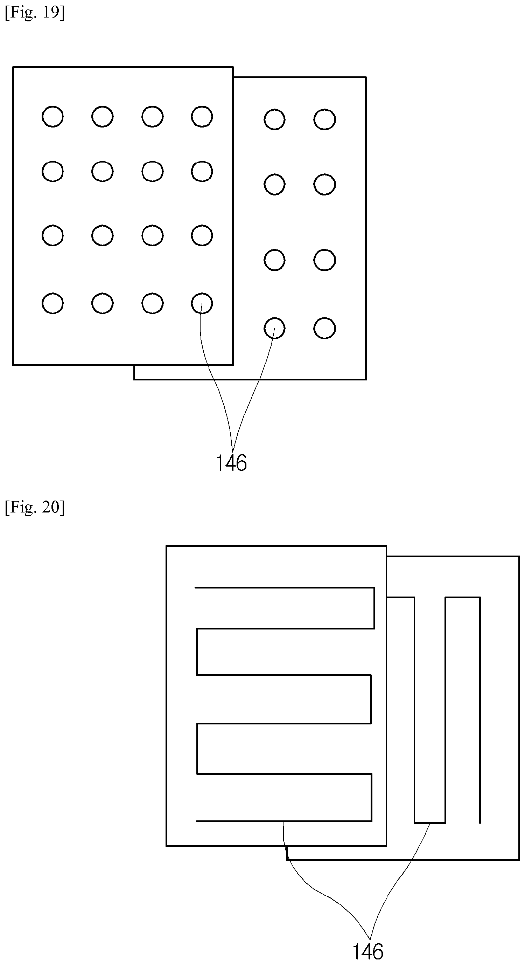

On the other hand, a following description will be given with respect to FIGS. 19 to 32.

In the embodiment of the present invention, as shown in FIG. 9, there may be further provided a radioactive concentration measuring sensor 191 positioned on the body part 110 and measuring radioactive concentration of the surrounding environment of a user. There may be provided a sound source output portion 192 positioned at the ear loop part 130 and transmitting information of the internal space environment of the body part 110 or the surrounding environment of a user in a voice form. Herein, the sound source output portion 192 may be a speaker, but is not limited thereto.

The radioactive concentration measuring sensor 191 measures the radioactive concentration of the surrounding environment of a user and transmits a result of measurement to a radioactivity measuring unit 316 of a controller shown in FIG. 12. The radioactivity measuring unit 316 determines whether the radioactive concentration of the surrounding environment of a user is within a preset allowable radioactive concentration range. When radioactive concentration exceeding the allowable concentration is detected, a warning is notified to a user by the sound source output portion 192. The preset allowable radioactive concentration range may be set differently depending on the type of nucleus.

The sound source output portion 192 may cooperate with other configurations of the environment measuring module 110. In this case, information of a current temperature in the mask, information of current gas concentration in the mask, and information of current fine dust concentration in the mask are transmitted in a voice form to the temperature measuring unit 111, the gas measuring unit 12, and the fine dust measuring unit 114, respectively.

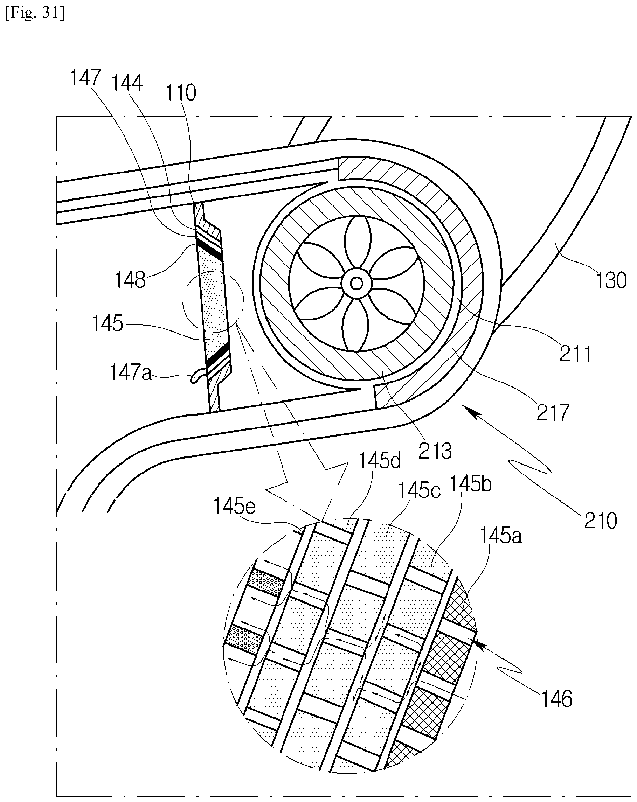

Furthermore, in the embodiment of the present invention, as shown in FIG. 31, another example of the filter part 140 may include a filter attachment/detachment portion 144, a multi-filter 145, and a filter block 147.

First, the filter attachment/detachment portion 144 may protrude slightly outward from the side of the body part 110. The multi-filter 145 may be positioned at the filter attachment/detachment portion 144 and may be comprised of multiple filter layers. The filter block 147 may be bonded to the multi-filter 145 by an adhesive member 148 and may be attached to and detached from the filter attachment/detachment portion 144. The filter block 147 may be provided at a side there of with a release handle 147a pulled by a user to release the multi-filter 145 so as to be easily removed.

Herein, the filter attachment/detachment portion 144 has a magnetic component in powder form that is applied thereto, and the filter block 147 may be made of a metal material that reacts with magnetism, such that the filter attachment/detachment portion and the filter block are attachable and detachable to and from each other by a magnetic force.

Furthermore, referring to an enlarged view of FIG. 31, the multi-filter 145 may be comprised of a total of five filter layers in the embodiment of the present invention. However, a single or multiple filter layers may be possible depending on the use environment, but is not limited thereto. The multi-filter 145 may be made of a material such as PolyCarbonate (PC), Tritan, PolyPropylene (PP), or the like.

The multi-filter 145 may include a first filter 145a, a second filter 145b, a third filter 145c, a fourth filter 145d, and a fifth filter 145e, which will be described limited to the embodiment of the present invention.

Prior to the description, it is assumed that the size of dust particles in the atmosphere causing respiratory disturbance is in a range of approximately 1 to 15 µm, the size of pollen particles causing allergies is approximately 1 to 60 µm, the size of viruses causing a lung disease is in a range of approximately 0.05 to 0.1 µm. Although not mentioned, foreign substances to be filtered out through the multi-filter 145 may vary.

The size of pores of each filter layer is determined depending on the size of foreign substances to be filtered out through each filter layer.

The first filter 145a is a part bonded to the filter block 147 and is a filter layer positioned at the outermost side of the filter attachment/detachment portion 144. The second filter 145b is positioned abutting the first filter 145a. The first filter 145a may be provided in a mesh shape and may have a pore size in a range of 0.02 to 59 µm, and the second filter 145b may have a pore size in a range of 0.01 to 59 µm, whereby foreign substances such as viruses, pollen, fine dust, and the like are filtered out.

The third filter 145c is positioned abutting the second filter 145b, and may have a pore size in a range of 0.01 to 59 µm and may contain a deodorizing component. Accordingly, filtering out foreign substances and deodorization are possible simultaneously.

The fourth filter 145d is positioned abutting the third filter 145c, and may have a pore size in a range of 0.02 to 59 µm and may contain an adsorbent material for removing toxic or harmful substances. Accordingly, air from which foreign substances are filtered out and deodorized is subjected to adsorption of toxic or harmful substances whereby more purified air is supplied into the mask.

The fifth filter 145e is positioned abutting the fourth filter 145d, and may have a pore size in a range of 0.01 to 1 mm and may contain an aroma component or a therapeutic component for a patient suffering from bronchial disease . The reason why the pore size of the fifth filter 145e is relatively large is to allow a drug component or aroma component normally having a large particle size to sufficiently pass through the filter to enter the user's respiratory organs.

Thus, in addition to filtering out foreign substances, an appropriate drug is allowed to be introduced into the mask to treat a patient who suffers from bronchial disease such as asthma, pneumonia, and the like. Alternatively, the aroma component such as an aromatic scent, a blueberry scent, or the like is allowed to be introduced into the mask, providing a comfortable feel to a user.

Herein, pores of respective filters constituting the multi-filter 145 may arranged alternately in a staggered arrangement as shown in the enlarged view of FIG. 13. The aim of this arrangement is to enhance the effect of filtering out foreign substances each time the foreign substances pass through each filter layer when the foreign substances in air flowing from the first filter 145a flow to the fifth filter 145e while flowing through the pores arranged alternately in the staggered arrangement.

Furthermore, pores of respective filters constituting the multi-filter 145 may be arranged in different patterns. FIGS. 19 to 21 show arrangement patterns of pores 146.

Referring to FIG. 19, multiple pores 146 are formed in a first filter paper to be arranged at a regular interval, and multiple pores 146 are formed in a second filter paper to be arranged at a regular interval at positions different from those of the first filter paper. Accordingly, air flows alternately while passing through the pores of the respective filter papers and thus a certain amount of foreign substances are filtered out through the filter papers.

FIG. 20 shows another arrangement pattern wherein a pore 146 is formed in the first filter paper in a zigzag pattern in a transverse direction, and a pore 146 is formed in the second filter paper in a zigzag pattern in a longitudinal direction. Accordingly, air also flows alternately while passing through the pores of the respective filter papers formed in a zigzag pattern and thus a certain amount of foreign substances are filtered out through the filter papers.

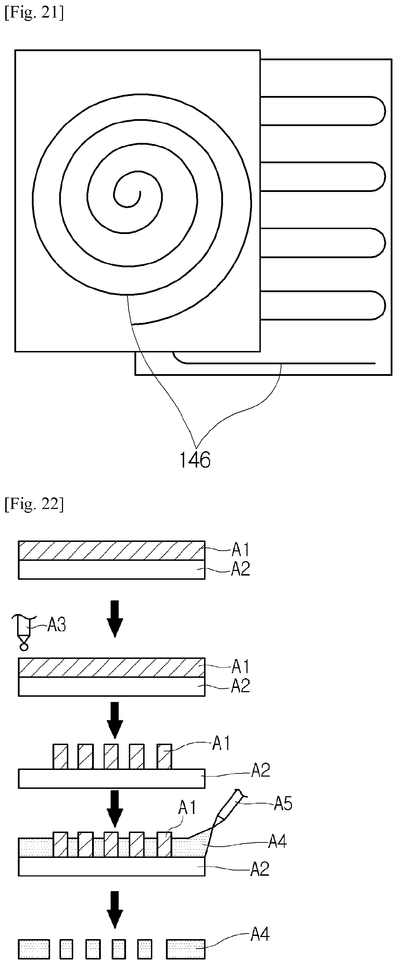

FIG. 21 shows still another arrangement pattern wherein a pore 146 is formed in the first filter paper in a circumferential direction, and a pore 146 is formed in the second filter paper in a zigzag pattern in a transverse direction. Accordingly, air also flows alternately while passing through the pores of the respective filters papers formed in the circumferential direction and in a zigzag pattern and thus a certain amount of foreign substances are filtered out through the filter papers.

Each of the arrangement patterns of the pores 146 shown in FIGS. 19 to 21 provides air flow direction diversity and air distribution, thereby enhancing the effect of filtering out foreign substances through each filter paper.

The arrangement patterns of the pores 146 may be applicable to all of the first filter 145a, the second filter 145b, the third filter 145c, the fourth filter 145d, and the fifth filter 145e that constitute the multi-filter 145.

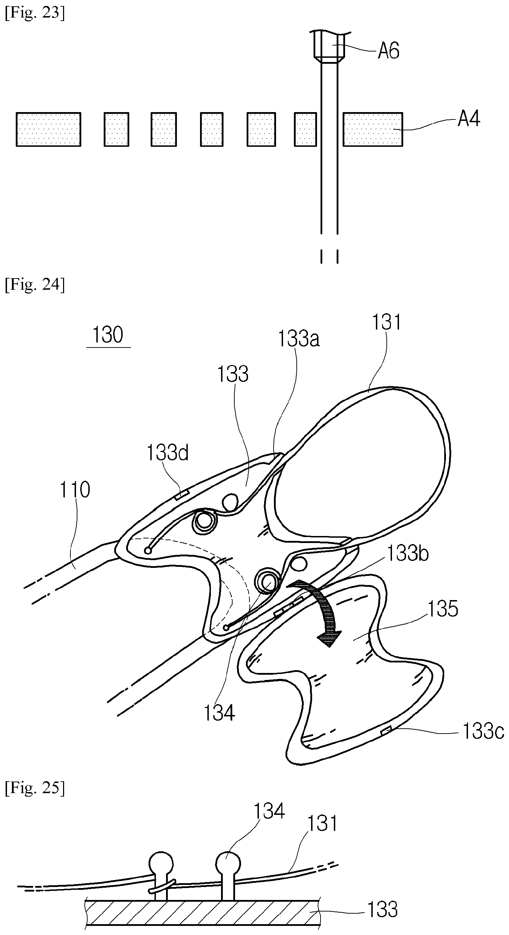

Next, FIGS. 22 and 23 show a method of manufacturing each filter layer of the multi-filter 145.

First, FIG. 23 shows a method of forming pores using femtosecond lasers (A6). In recent years, formation of nanomaterials using the femtosecond lasers has been gradually increasing and thus may be suitable for forming the pores of the filter layers according to the present invention, which are formed in a nano unit. The formation of pores using the femtosecond lasers ensures pores with clean edges.

Next, FIG. 22 shows a method of forming pores using etching. In a first step, an etching material A1 is placed on a work piece A2, and in a second step, an etching solution A3 such as hydrochloric acid, sulfuric acid, or the like is dropped at a predetermined interval or in a predetermined pattern. Herein, an automated machine may be used for precision mold making.

When the etching solution A3 is dropped on the etching material A1 and the etching is completed, a mold having a predetermined interval or a predetermined pattern is formed in the etching material A1 as in a third step. In a fourth step, a filter resin A4 is injected into the mold A1 of the etching material through an injection nozzle A5. After a certain period of time, as in a fifth step, pores having a predetermined interval or a predetermined pattern are formed in the filter resin A4.

On the other hand, FIGS. 24 to 26 show various examples of the ear loop part 130 according to the present invention.

First, referring to FIGS. 24 and 25, the ear loop part 130 may include a strip 131 and a length adjusting unit 132.

The strip 131 is connected to the side of the body part 110 and is looped around the user's ear and is configured such that the mask can be tightly fitted to the user's face. The strip 131 may be made of an elastic fiber material, but is not limited thereto.

The length adjusting unit 132 is positioned at the side of the body part 110 and is connected with the strip 131 and is provided to adjust the length of the strip 131. The length adjusting unit 132 may include a bending body 133, a bending cover 135, and a bending protrusion 134.

The bending body 133 may be a portion that is securely positioned at the side of the body part 110. Furthermore, the bending cover 135 may be connected at a first side thereof to the bending body 133 by a hinge 133b and may be connected at a second side thereof to the bending body 133 to be attachable and detachable thereto and therefrom by magnetic members 133c and 133d. The bending cover may be configured to be openable and closeable. The bending body 133 has an opening 133a formed therein, such that the strip 131 secured to the side of the body part 110 may partially protrude to the outside of the bending body.

Herein, the bending protrusion 134 is positioned on the bending body 133 and may be provided to adjust the length of the strip 131 by allowing the strip 131 to be wound thereon. In FIG. 24, it can be seen that four bending protrusions 134 are arranged in pairs such that two bending protrusions in each pair are positioned at opposite sides of the bending body 133, respectively. A user can wind the strip 131 on the bending protrusions 134 to adjust the length of the strip 131 that is looped around the user's ear. When the strip 131 is adjusted in length such that the strip 131 is tightly looped around the user's ear, the mask conforms to the facial skeleton of a user so as to be tightly fitted thereto. Thus, it is possible to stably block entry of external air into the mask, except for the filter part 140.

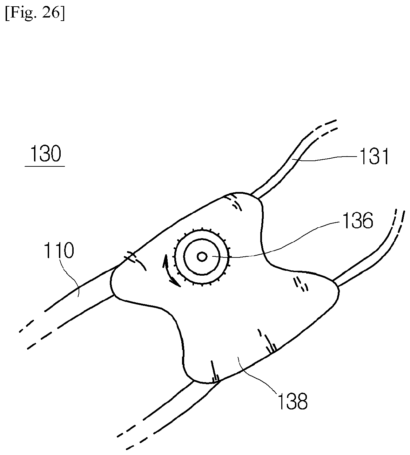

Next, In FIG. 26, a reel type dial 136 positioned at an outer surface of a bending housing 138 that is secured to the side of the body part 110 is shown as another example for adjusting the length of the strip 131. A user can adjust the length of the strip 131 by turning the dial 136 in a first direction or in a second direction.

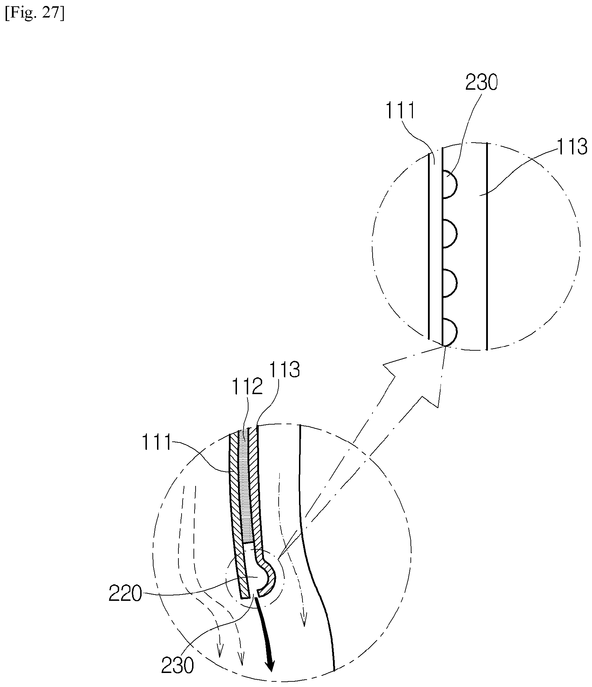

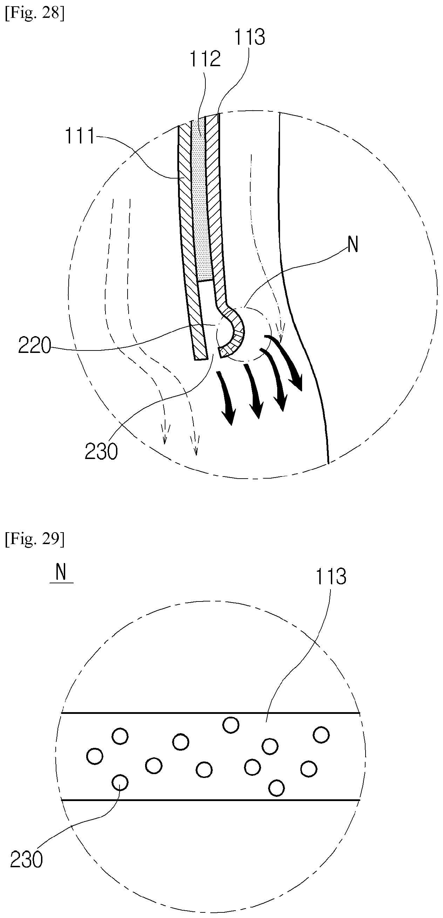

On the other hand, FIGS. 27 and 28 show another example of structures of the first air passage 220 and the air nozzle 230.

First, referring to FIG. 27, the outer surface 111,the intermediate surface 112, and the inner surface 113 of the body part 110 are arranged in order such that the intermediate surface 112 is bonded to the inside of the outer surface 111 and the inner surface is bonded to the inside of the intermediate surface 112.

Herein, the intermediate surface 112 is shorter than the outer surface 111 and the inner surface 113 in length such that an end thereof is positioned inwardly of ends of the outer and inner surfaces. Additionally, the end of the inner surface 113 may be formed in a slightly rounded shape to define a space where purified air can flow. Due to this structure, the air nozzle 230 can be formed naturally by an interface between the outer surface 111 and the inner surface 113.

Herein, the air nozzle 230 communicates with the first air passage 220 and multiple air nozzles may be formed at the lower edge of the body part 110, such that the air curtain is formed along the skeletal contours of the peripheral portion of the user's chin or cheeks, thereby blocking entry of external substances.

In other words, as shown in an enlarged view of FIG. 27, multiple round recesses are formed on the inner surface 113 of the body part 110 to be arranged in a direction along the chin contour, and the round recesses are bonded to the outer surface 111 of the body part 110, thereby forming the multiple air nozzles 230.

Although not shown in the drawings, a slightly round recess may be formed on the outer surface 111 such that the recess is in contact with each of the round recesses formed on the inner surface 113, thereby forming a circular air nozzle 230.

Next, referring now to FIG. 28, multiple air nozzle 30 may be formed at a distal end of the inner surface 113 of the body part 110 as well as being defined by the ends of the outer surface 111 and the inner surface 113 of the body part 110, such that an injection area of the filtered air can be expanded.

In other words, a part of the filtered air is injected to the chin in an oblique direction, thereby enhancing the effect of the fluid barrier together with the filtered air injected toward the lower edge of the body part.

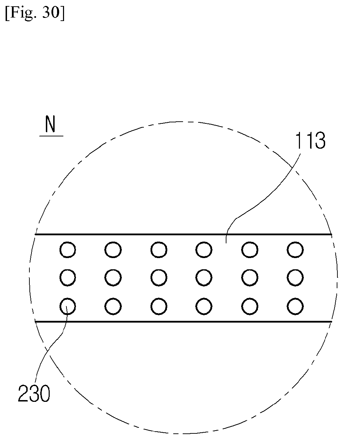

The arrangement of the multiple air nozzles 230 is shown in FIGS. 29 and 30. First, FIG. 29 shows a state in which the multiple air nozzles 230 are irregularly arranged at the distal end of the inner surface 113 of the body part 110. Due to this irregular arrangement, a turbulent flow is generated at the peripheral portion of the user's chin, thereby forming a fluid barrier.

FIG. 30 shows a state in which the multiple air nozzles 230 are regularly arranged at the distal end of the inner surface 113 of the body part 110. Due to this regular arrangement, the filtered air flows along the contours of the user's chin, thereby forming a fluid barrier having a relatively uniform boundary layer.

More specifically, the filtered air having flowed along the first air passage 220 is injected toward a lower portion of the body part 110 through the multiple air nozzles 230, and then flows along the contours of the user' chin to stay attached thereto.

Herein, an air curtain is formed, which causes the Coandǎ effect. Herein, the Coandǎ effect is a phenomenon in which a jet flow attaches itself to a nearby surface and remains attached.

Due to the Coandǎ effect, a user's exhaled carbon dioxide and external harmful substances are swept downward together with the air curtain to form a fluid barrier, thereby blocking entry of unfiltered substances into the mask.

Herein, because the lower edge of the body part 110 is a portion that is not tightly fitted to the skin but exerts an effect of being indirectly fitted to the skin using an air curtain, free movement of the user's chin can be permitted. Due to this function, a user can communicate clearly with others even when wearing the mask.

Next, FIG. 32 shows another example of the air blowing member and the filter part. A fan cover 212 is positioned to protrude from the outer surface 111 of the body part 110, and a space through which air can flow is defined inside the fan cover 212.

Furthermore, a fan seat 211 is formed on the outer surface of the body part 110 at a position inside the fan cover 212, and the blowing fan 213 is positioned on the fan seat 211. Furthermore, the body part 110 may have a spacing space defined between the outer surface 111 and the intermediate surface 112, and the multi-filter 145 may be positioned in the spacing space to be bonded together therewith.

Furthermore, the intermediate surface 112 and the inner surface 113 of the body part 110 are coupled to be spaced apart from each other by a predetermined distance and thus the first air passage 220 is defined therebetween. This structure is also applicable to a structure defining the second air passage 240.

Due to this structure, external air flows into the space inside the fan cover 212, passes through the blowing fan 213, and then is filtered through the multi-filter 145, thereby removing foreign substances. Thereafter, a part of the filtered air is injected to the user's chin through the first air passage 220 to cause the Coandǎ effect to occur while a remaining part of the filtered air is supplied to the user's respiratory organs through the second air passage 240 to help a user respire.

Meanwhile, in the embodiment of the present invention, a filter module that detects the gas component and concentration of exhalation from the user's respiratory organs may be mounted inside or outside the mask.

Furthermore, as shown in FIG. 9, there may be provided a biomarker unit 180, which may be provided in the form of a mounting type biochip, such as a lab-on-a-chip recognizing a particular material, or may be provided in the form of an application type biomarker material changing in color in response to a specific substance, thereby being used to ascertain or diagnose the presence or absence of foreign substances.

Although a preferred embodiments of the present invention has been described for illustrative purposes, those skilled in the art will appreciate that various modifications, additions and substitutions are possible, without departing from the scope of the invention as disclosed in the accompanying claims.

### Industrial Applicability

The present invention relates to a health mask.

## Claims

1. A health mask, comprising:
a body part (110) resting on and covering a partial area of a face, including a respiratory system of a user, the body part (110) having an internal space formed therein;
a tight fitting part (120) positioned along a circumference of the body part (110) such that the body part (110) is tightly fitted to the partial area of the face;
an ear loop part (130) positioned at opposite sides of the body part (110) such that the body part (110) is secured to the partial area of the face the health mask being **characterized by**:
a filter part (140) positioned at the opposite sides of the body part (110) such that external air is purified and supplied to the internal space of the body part (110); and
an air curtain-forming means (200) provided on the body part (110) such that an air curtain is formed around the body part (110) to block entry of external substances.

2. The health mask of claim 1, wherein the air curtain-forming means (200) includes:
an air blowing member (210) provided on the body part (110) at a position between the filter part (140) and the ear loop part (130);
a first air passage (220) positioned in the internal space of the body part (110) to allow the air blowing member (210) and a peripheral portion of a user's chin to communicate with each other, such that filtered air is supplied to the peripheral portion of the user's chin; and
multiple nozzles communicating with the first air passage (220) and formed at a lower edge of the body part (110), such that the air curtain is formed along skeletal contours of the user's chin to block the entry of the external substances.

3. The health mask of claim 2, wherein the air curtain-forming means (200) further includes:
a second air passage (240) positioned in the internal space of the body part (110) to allow the air blowing member (210) and a peripheral portion of a user's nose to communicate with each other, such that the filtered air is supplied to the peripheral portion of the user's nose.

4. The health mask of claim 3, wherein the first air passage (220) is greater than the second air passage (240) in diameter.

5. The health mask of claim 2, wherein the air blowing member (210) includes:
a fan seat (211) provided on the body part (110) at the position between the filter part (140) and the ear loop part (130);
a blowing fan (213) positioned outside the fan seat (211) and supplying the filtered air flowing from the filter part (140) to the internal space of the body part (110); and
a battery unit (215) positioned inside the fan seat (211) and connected to the blowing fan (213), the battery unit (215) being provided to supply electric power to the blowing fan (213).

6. The health mask of claim 5, wherein the air blowing member (210) further includes:
a sound insulation unit (217) positioned on the body part (110) at a position between the fan seat (211) and the ear loop part (130) for sound insulation of the blowing fan (213).

7. The health mask of claim 5, further comprising:
a medium member (170) positioned on the body part (110) at a position between the filter part (140) and the blowing fan (213), such that a medium containing an aroma or drug is contained in the filtered air.

8. The health mask of claim 1, wherein the tight fitting part (120) is provided with:
an anti-loosening unit (121) positioned at a portion of the tight fitting part (120) that is tightly fitted to a bridge of a nose, such that the body part (110) is prevented from loosening downward along contours of the bridge of the nose, wherein
the anti-loosening unit (121) is made of nanofibers obtained by producing multiple fine bristles by injection molding.

9. The health mask of claim 8, wherein the multiple fine bristles are inclined upward and arranged in multiple stages in a vertical direction.

10. The health mask of claim 1, wherein the tight fitting part (120) is made of a shape memory resin that contracts according to a user's body temperature and is deformed to conform to skeletal contours of the user's face.

11. The health mask of claim 5, further comprising:
a temperature adjusting unit (160) cooperating with the filter part (140) such that the air flowing to the internal space of the body part (110) is adjusted in temperature, wherein
the temperature adjusting unit (160) includes:
a temperature measuring sensor (161) positioned at a center of the body (110) part; and
a heating unit (162) positioned on the body part (110) at a position between the filter part (140) and the blowing fan (213) such that the air flowing from the filter part (140) is heated, the heating unit (162) being connected with the battery unit (215).

12. The health mask of claim 1, further comprising:
a goggle part (410) assembled with ear loop parts (130) and the body part (110) such that user's eyes are protected.

13. The health mask of claim 12, further comprising:
an image capturing unit (420) positioned on the ear loop part (130) at a position abutting a side of the goggle part (410) such that atmospheric environment or a user's work environment is captured; and
a heat sensor (430) positioned on the ear loop part (130) at the position abutting the side of the goggle part (410) such that a living creature in a user's surrounding area is sensed.

14. The health mask of claim 12, further comprising:
an image capturing unit (420) positioned on the ear loop part (130) at a position abutting a side of the goggle part (410) such that atmospheric environment or a user's work environment is captured; and
a lighting unit (460) positioned on the ear loop part (130) at the position abutting the side of the goggle part (410) such that user's view in a user's surrounding area is secured.

15. The health mask of claim 1, wherein a biomarker unit (180) is positioned on an inner surface (113) of the body part (110) or on the filter part (140).

## Patentansprüche

1. Eine Gesundheitsmaske, welche folgendes aufweist:
ein Körperteil (110), das auf einem Teilbereich eines Gesichts aufliegt und diesen bedeckt, einschließlich einem Atmungssystem eines Anwenders, wobei das Körperteil (110) einen darin gebildeten Innenraum hat;
ein eng sitzendes Teil (120), das entlang eines Umfangs des Körperteils (110) derart positioniert ist, dass das Körperteil (110) eng an dem Teilbereich des Gesichts sitzt;
ein Ohrschlaufenteil (130), das an gegenüberliegenden Seiten des Körperteils (110) derart positioniert ist, dass das Körperteil (110) an dem Teilbereich des Gesichts befestigt ist, wobei die Gesundheitsmaske durch folgendes gekennzeichnet ist:
ein Filterteil (140), das an den gegenüberliegenden Seiten des Körperteils (110) derart positioniert ist, dass Außenluft gereinigt und zu dem Innenraum des Körperteils (110) geliefert wird; und
Luftvorhangbildemittel (200), die auf dem Körperteil (100) derart vorgesehen sind, dass ein Luftvorhang um das Körperteil (110) herum gebildet ist, um das Eindringen von äußeren Substanzen zu blockieren.

2. Die Gesundheitsmaske nach Anspruch 1, wobei die Luftvorhangbildemittel (200) folgendes aufweisen:
ein Luftgebläseglied (210), das auf dem Körperteil (110) an einer Position zwischen dem Filterteil (140) und dem Ohrschlaufenteil (130) vorgesehen ist;
einen ersten Luftdurchlass (220), der in dem Innenraum des Körperteils (110) positioniert ist, um dem Luftgebläseglied (210) und einem peripheren Teil eines Kinns eines Anwenders zu gestatten, miteinander derart zu kommunizieren, dass gefilterte Luft zu dem peripheren Teil des Kinns des Anwenders geliefert wird; und
mehrere Düsen, die mit dem ersten Luftdurchlass (220) kommunizieren und an einer unteren Kante des Körperteils (110) derart gebildet sind, dass der Luftvorhang entlang von Skelettkonturen des Kinns des Anwenders gebildet ist, um den Eintritt der äußeren Substanzen zu blockieren.

3. Die Gesundheitsmaske nach Anspruch 2, wobei die Luftvorhangbildemittel (200) ferner folgendes aufweisen:
einen zweiten Luftdurchlass (240), der in dem Innenraum des Körperteils (110) positioniert ist, um es dem Luftgebläseglied (210) und einem peripheren Teil einer Nase eines Anwenders zu gestatten, miteinander derart zu kommunizieren, dass die gefilterte Luft zu dem peripheren Teil der Nase des Anwenders geliefert wird.

4. Die Gesundheitsmaske nach Anspruch 3, wobei der erste Luftdurchlass (220) im Durchmesser größer als der zweite Luftdurchlass (240) ist.

5. Die Gesundheitsmaske nach Anspruch 2, wobei das Luftgebläseglied (210) folgendes aufweist:
einen Lüftersitz (211), der auf dem Körperteil (110) an der Position zwischen dem Filterteil (140) und dem Ohrschlaufenteil (130) vorgesehen ist;
ein Gebläselüfter (213), der außerhalb des Lüftersitzes (211) positioniert ist und die gefilterte Luft, welche von dem Filterteil (140) zu dem Innenraum des Körperteils (100) strömt, liefert; und
eine Batterieeinheit (215), die innerhalb des Lüftersitzes (211) positioniert und mit dem Gebläselüfter (213) verbunden ist, wobei die Batterieeinheit (215) vorgesehen ist, um elektrische Leistung zu dem Gebläselüfter (213) zu liefern.

6. Die Gesundheitsmaske nach Anspruch 5, wobei das Luftgebläseglied (210) ferner folgendes aufweist:
eine Schallisolierungseinheit, die auf dem Körperteil (110) an einer Position zwischen dem Lüftersitz (211) und dem Ohrschlaufenteil (130) für die Schallisolierung des Gebläselüfters (213) positioniert ist.

7. Die Gesundheitsmaske nach Anspruch 5, welche ferner folgendes aufweist:
ein Mediumglied (170), das auf dem Körperteil (110) an einer Position zwischen dem Filterteil (140) und dem Gebläselüfter (213) derart positioniert ist, dass ein Medium, welches einen Duft oder Arzneimittel enthält, in der gefilterten Luft enthalten ist.

8. Die Gesundheitsmaske nach Anspruch 1, wobei das eng sitzende Teil (120) mit folgendem versehen ist:
eine Anti-Lockerungs-Einheit (121), die an einem Teil des eng sitzenden Teils (120), das eng an einen Nasenrücken befestigt ist, derart positioniert ist, dass der Körperteil (110) daran gehindert wird, sich entlang der Konturen des Nasenrückens nach unten zu lockern, wobei
die Anti-Lockerungs-Einheit (121) aus Nanofasern hergestellt ist, die durch Produktion mehrerer feiner Borsten durch Einspritzgießen erhalten werden.

9. Die Gesundheitsmaske nach Anspruch 8, wobei die mehreren feinen Borsten nach oben geneigt und in mehreren Stufen in einer vertikalen Richtung angeordnet sind.

10. Die Gesundheitsmaske nach Anspruch 1, wobei das eng sitzende Teil (120) aus einem Formgedächtnisharz hergestellt ist, das sich gemäß einer Körpertemperatur eines Anwenders zusammenzieht, und verformt ist, um sich an die Skelettkonturen des Gesichts des Anwenders anzupassen.

11. Die Gesundheitsmaske nach Anspruch 5, welche ferner folgendes aufweist:
eine Temperatureinstelleinheit (160), die mit dem Filterteil (140) derart zusammenarbeitet, dass die Luft, die in den Innenraum des Körperteils (110) strömt, in der Temperatur eingestellt wird, wobei
die Temperatureinstelleinheit (160) folgendes aufweist:
einen Temperaturmesssensor (161), der an einer Mitte des Körperteils (110) positioniert ist; und
eine Heizeinheit (162), die auf dem Körperteil (110) an einer Position zwischen dem Filterteil (140) und dem Gebläselüfter (213) derart positioniert ist, dass die Luft, welche von dem Filterteil (140) strömt, erhitzt wird, wobei die Heizeinheit (162) mit der Batterieeinheit (215) verbunden ist.

12. Die Gesundheitsmaske nach Anspruch 1, welche ferner folgendes aufweist:
ein (Schutz-)brillenteil (410), das mit Ohrschlaufenteilen (130) und dem Körperteil (110) derart zusammengebaut ist, dass Augen des Anwenders geschützt sind.

13. Die Gesundheitsmaske nach Anspruch 12, welche ferner folgendes aufweist:
eine Bilderfassungseinheit (420), die auf dem Ohrschlaufenteil (130) an einer Position, die an einer Seite des (Schutz-)brillenteils (410) derart anliegt, dass die atmosphärische Umgebung oder eine Arbeitsumgebung eines Anwenders erfasst wird, positioniert ist; und
einen Wärmesensor (430), der auf dem Ohrschlaufenteil (130) an der Position, die an der Seite des (Schutz-)brillenteils (410) anliegt, derart positioniert ist, dass ein lebendes Wesen in dem umgebenden Bereich eines Anwenders erfasst wird.

14. Die Gesundheitsmaske nach Anspruch 12, welche ferner folgendes aufweist:
eine Bilderfassungseinheit (420), die auf dem Ohrschlaufenteil (130) an einer Position, die an einer Seite des (Schutz-)brillenteils (410) anliegt, derart positioniert ist, dass die atmosphärische Umgebung oder eine Arbeitsumgebung eines Anwenders erfasst wird; und
eine Beleuchtungseinheit (460), die auf dem Ohrschlaufenteil (130) an der Position, die an der Seite des (Schutz-)brillenteils (410) anliegt, derart positioniert ist, dass eine Sicht des Anwenders in einem umgebenden Bereich eines Anwenders gesichert ist.

15. Die Gesundheitsmaske nach Anspruch 1, wobei eine Biomarkereinheit (180) auf einer Innenoberfläche (113) des Körperteils (110) oder auf dem Filterteil (140) positioniert ist.

## Revendications

1. Masque hygiénique, comprenant :
une partie corps (110) reposant sur une zone partielle d'un visage et couvrant celle-ci, incluant un système respiratoire d'un utilisateur, la partie corps (110) ayant un espace interne formé à l'intérieur ;
une partie à ajustement étroit (120) positionnée le long d'une circonférence de la partie corps (110) de telle sorte que la partie corps (110) soit ajustée étroitement à la zone partielle du visage ;
une partie boucles d'oreilles (130) positionnée en des côtés opposés de la partie corps (110) de telle sorte que la partie corps (110) soit immobilisée sur la zone partielle du visage, le masque hygiénique étant **caractérisé par** :
une partie filtre (140) positionnée en les côtés opposés de la partie corps (110) de telle sorte que de l'air externe soit purifié et amené jusqu'à l'espace interne de la partie corps (110) ; et
des moyens formant rideau d'air (200) prévus sur la partie corps (110) de telle sorte qu'un rideau d'air soit formé autour de la partie corps (110) pour bloquer l'entrée de substances externes.

2. Masque hygiénique selon la revendication 1, dans lequel les moyens formant rideau d'air (200) incluent :
un organe de projection d'air (210) prévu sur la partie corps (110) en une position entre la partie filtre (140) et la partie boucles d'oreilles (130) ;
un premier passage d'air (220) positionné dans l'espace interne de la partie corps (110) pour permettre à l'organe de projection d'air (210) et à une partie périphérique d'un menton d'utilisateur de communiquer entre eux, de telle sorte que de l'air filtré soit amené jusqu'à la partie périphérique du menton d'utilisateur ; et
de multiples buses communiquant avec le premier passage d'air (220) et formées en un bord inférieur de la partie corps (110), de telle sorte que le rideau d'air soit formé le long de contours squelettiques du menton d'utilisateur pour bloquer l'entrée des substances externes.

3. Masque hygiénique selon la revendication 2, dans lequel les moyens formant rideau d'air (200) incluent en outre :
un deuxième passage d'air (240) positionné dans l'espace interne de la partie corps (110) pour permettre à l'organe de projection d'air (210) et à une partie périphérique d'un nez d'utilisateur de communiquer entre eux, de telle sorte que l'air filtré soit amené jusqu'à la partie périphérique du nez d'utilisateur.

4. Masque hygiénique selon la revendication 3, dans lequel le premier passage d'air (220) est plus grand que le deuxième passage d'air (240) en diamètre.

5. Masque hygiénique selon la revendication 2, dans lequel l'organe de projection d'air (210) inclut :
un siège de ventilateur (211) prévu sur la partie corps (110) en la position entre la partie filtre (140) et la partie boucles d'oreilles (130) ;
un ventilateur (213) positionné à l'extérieur du siège de ventilateur (211) et amenant l'air filtré s'écoulant depuis la partie filtre (140) jusqu'à l'espace interne de la partie corps (110) ; et
une unité de batterie (215) positionnée à l'intérieur du siège de ventilateur (211) et reliée au ventilateur (213), l'unité de batterie (215) étant prévue pour fournir de l'énergie électrique au ventilateur (213).

6. Masque hygiénique selon la revendication 5, dans lequel l'organe de projection d'air (210) inclut en outre :
une unité d'isolation phonique (217) positionnée sur la partie corps (110) en une position entre le siège de ventilateur (211) et la partie boucles d'oreilles (130) pour une isolation phonique du ventilateur (213).

7. Masque hygiénique selon la revendication 5, comprenant en outre :
un organe de médium (170) positionné sur la partie corps (110) en une position entre la partie filtre (140) et le ventilateur (213), de telle sorte qu'un médium contenant un arôme ou un médicament soit contenu dans l'air filtré.

8. Masque hygiénique selon la revendication 1, dans lequel la partie à ajustement étroit (120) est pourvue :
d'une unité anti-desserrement (121) positionnée en une partie de la partie à ajustement étroit (120) qui est ajustée étroitement sur une arête d'un nez, de telle sorte que la partie corps (110) soit empêchée de se desserrer vers le bas le long de contours de l'arête du nez, dans lequel
l'unité anti-desserrement (121) est faite de nanofibres obtenues en produisant de multiple crins fins par moulage par injection.

9. Masque hygiénique selon la revendication 8, dans lequel les multiples crins fins sont inclinés vers le haut et arrangés en de multiples étages dans une direction verticale.

10. Masque hygiénique selon la revendication 1, dans lequel la partie à ajustement étroit (120) est faite d'une résine à mémoire de forme qui se contracte selon une température du corps de l'utilisateur et est déformée pour s'adapter aux contours squelettiques du visage de l'utilisateur.

11. Masque hygiénique selon la revendication 5, comprenant en outre :
une unité d'ajustement de température (160) coopérant avec la partie filtre (140) de telle sorte que l'air s'écoulant jusqu'à l'espace interne de la partie corps (110) soit ajusté en température, dans lequel
l'unité d'ajustement de température (160) inclut :
un capteur de mesure de température (161) positionné en un centre de la partie corps (110) ; et
une unité de chauffage (162) positionnée sur la partie corps (110) en une position entre la partie filtre (140) et le ventilateur (213), de telle sorte que l'air s'écoulant depuis la partie filtre (140) soit chauffé, l'unité de chauffage (162) étant reliée à l'unité de batterie (215).

12. Masque hygiénique selon la revendication 1, comprenant en outre :
une partie lunettes (410) assemblée avec les parties boucles d'oreilles (130) et à la partie corps (110) de telle sorte que les yeux de l'utilisateur soient protégés.

13. Masque hygiénique selon la revendication 12, comprenant en outre :
une unité de capture d'image (420) positionnée sur la partie boucles d'oreilles (130) en une position adjacente à un côté de la partie lunettes (410) de telle sorte que l'environnement atmosphérique ou un environnement de travail de l'utilisateur soit capturé ; et
un capteur de chaleur (430) positionné sur la partie boucles d'oreilles (130) en la position adjacente au côté de la partie lunettes (410) de telle sorte qu'une créature vivante dans une zone environnante de l'utilisateur soit captée.

14. Masque hygiénique selon la revendication 11, comprenant en outre :
une unité de capture d'image (420) positionnée sur la partie boucles d'oreilles (130) en une position adjacente à un côté de la partie lunettes (410) de telle sorte que l'environnement atmosphérique ou un environnement de travail de l'utilisateur soit capturé ; et
une unité d'éclairage (460) positionnée sur la partie boucles d'oreilles (130) en la position adjacente au côté de la partie lunettes (410) de telle sorte que la vue de l'utilisateur dans une zone l'entourant soit garantie.

15. Masque hygiénique selon la revendication 1, dans lequel une unité à biomarqueur (180) est positionnée sur une surface intérieure (113) de la partie corps (110) ou sur la partie filtre (140).
